(19) Europäisches Patentamt
European Patent Office
Office européen des brevets

(11) **EP 4 692 353 A1**

(12) **EUROPEAN PATENT APPLICATION**
published in accordance with Art. 153(4) EPC

(43) Date of publication:
**11.02.2026 Bulletin 2026/07**

(21) Application number: **24780598.9**

(22) Date of filing: **28.03.2024**

(51) International Patent Classification (IPC):
*C12N 15/57* (2006.01)   *C12N 1/15* (2006.01)
*C12N 1/19* (2006.01)   *C12N 1/21* (2006.01)
*C12N 5/10* (2006.01)   *C12N 9/48* (2006.01)
*C12Q 1/37* (2006.01)

(52) Cooperative Patent Classification (CPC):
**C12N 5/10; C12N 9/48; C12N 15/74; C12N 15/80;
C12N 15/81; C12Q 1/37**

(86) International application number:
**PCT/JP2024/012571**

(87) International publication number:
**WO 2024/204497 (03.10.2024 Gazette 2024/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **30.03.2023 JP 2023055723**

(71) Applicant: **Kikkoman Corporation
Noda-shi, Chiba 278-8601 (JP)**

(72) Inventors:
• **ICHIYANAGI Yuko
Noda-shi, Chiba 278-8601 (JP)**
• **ISHIKAWA Junya
Noda-shi, Chiba 278-8601 (JP)**
• **ICHIYANAGI Atsushi
Noda-shi, Chiba 278-8601 (JP)**

(74) Representative: **Mewburn Ellis LLP
Aurora Building
Counterslip
Bristol BS1 6BX (GB)**

Remarks:
The complete document including Reference Table(s) and the Sequence Listing(s) can be downloaded from the EPO website

(54) **RECOMBINANT PROTEIN**

(57) The present disclosure provides a means that enables detection of endotoxin. The present disclosure also provides a reagent for detecting endotoxin with thermostability. The present disclosure provides a recombinant Factor C protein having a given amino acid sequence, a polynucleotide encoding the same, a reagent for detecting endotoxin comprising the same, and a method for producing the same. The present disclosure also provides a recombinant Factor C protein having given amino acid substitution, a polynucleotide encoding the same, a reagent for detecting endotoxin comprising the same, and a method for producing the same.

**Description**

Technical Field

The present invention relates to a recombinant protein. More specifically, the present invention relates to, for example, a recombinant protein used to measure endotoxin.

Background Art

Endotoxin is a lipopolysaccharide constituting the cell wall of Gram-negative bacteria. Since endotoxin is a very stable substance, it is difficult to remove or inactivate endotoxin once it is included during a process of producing or using pharmaceutical products, medical equipment, dialysates, and the like. Endotoxin is a typical pyogenic substance. If the blood is contaminated with a very small amount (of the order of ng or pg) of endotoxin due to administration of a substance contaminated with endotoxin, endotoxin is known to develop pyrexia in a human or animal to which such substance has been administered and cause highly lethal diseases, such as intravascular blood coagulation associated with sepsis. Therefore, in the fields of injection preparations, medical equipment, biopharmaceuticals, regenerative medical products, medications for dialysis, and the like, it is critical that products do not contain endotoxin (pyrogen-free), and there is a market need for development of a technique that enables rapid and accurate measurement of endotoxin determination.

At present, the Limulus test is a mainstream technique to measure endotoxin. The Limulus test is performed based on the phenomenon such that a limulus amebocyte lysate (hereafter, also referred to as "LAL") reacts with endotoxin, and techniques, such as gelation method, turbidimetry method, colorimetry method, and fluorometry method, have been known (see, for example, Patent literatures 1 and 2). LAL-based reagents for measurement are denoted as LAL reagents, lysate reagents, limulus reagents, and the like, and these terms are mutually synonymous.

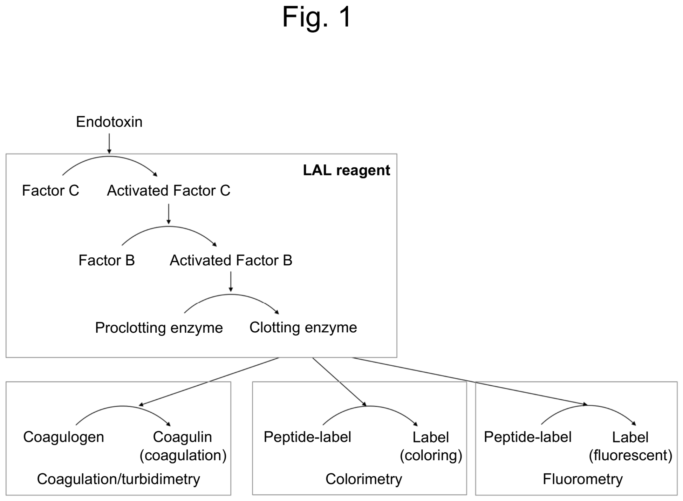

The principle for endotoxin measurement using LAL is as shown in the schematic diagram of Figure 1. Endotoxin convers Factor C as an enzyme precursor into activated Factor C. The activated Factor C functions as a hydrolase to convert Factor B as an enzyme precursor into activated Factor B. The activated Factor B is also a hydrolase, which converts a proclotting enzyme into an activated clotting enzyme. A clotting enzyme is a hydrolase. There is a method of measurement comprising hydrolyzing a synthetic substrate, such as a peptide-label, with the use of this hydrolytic activity and measuring the generated product or quenched product by the turbidimetry method, the chromogenic substrate method, the fluorescent substrate method, the luminescent substrate method, or the like.

Examples of genera of horseshoe crabs include the genera *Limulus, Tachypleus,* and *Carcinoscorpius.* Examples of horseshoe crab species include *Limulus polyphemus, Tachypleus tridentatus*, *Tachypleus gigas,* and *Carcinoscorpius rotundicauda.* A limulus amebocyte lysate is a finite bioresource. Since demand for LAL reagents is increasing, it is not preferable to continuously rely on the limulus amebocyte lysate as a supply source of LAL reagents from the viewpoint of sustainability.

Patent literature 3 discloses a recombinant protein derived from horseshoe crabs and DNA encoding the same. Patent literatures 4 to 11 each disclose a recombinant protein.

Non Patent literature 1 describes recombinant Factor C, which is a synthetic alternative to a limulus amebocyte lysate for detecting endotoxin aimed at horseshoe crab conservation. Although recombinant Factor C is commercialized, the authors of Non Patent literature 1 indicate, as the reason why switching from the LAL reagent to recombinant Factor C has not been advanced, that the disclosed recombinant Factor C is protected by an exclusive patent and the pharmaceutical industry has been reluctant to prepare reagents for detecting endotoxin by relying on a single supplier. Accordingly, the existence of known a recombinant Factor C does not mean other options are unnecessary.

Reagents for detecting endotoxin that have a low burden on the environment and can be stably supplied are in need. In addition, thermostable reagents for detecting endotoxin are in need.

Citation List

Patent Literature

Patent Literature 1: WO 1995/014931
Patent Literature 2: JP 2009-150903 A
Patent Literature 3: WO 2018/074498 (JP Patent No. 6,927,993)
Patent Literature 4: US Patent No. 5,712,144
Patent Literature 5: US Patent No. 5,716,834
Patent Literature 6: US Patent No. 5,858,706

Patent Literature 7: US Patent No. 5,985,590
Patent Literature 8: US Patent No. 6,645,724
Patent Literature 9: WO 2008/004674
Patent Literature 10: JP 2014-510898 A
Patent Literature 11: WO 2014/092079

Non Patent Literature

Non Patent Literature 1: PLoS Biol. 2018 Oct; 16(10): e2006607.

Summary of Invention

Technical Problem

In a particular embodiment, an object of the present disclosure is to provide a recombinant Factor C protein that is different from known Factor C proteins that would address at least some of the conventional problems including stable supply, which is an issue of concern for the pharmaceutical industry.
Further, in a particular embodiment, an object of the present disclosure is to provide a recombinant Factor C protein with thermostability.

Solution to Problem

The present inventors have conducted concentrated studies in order to attain the above objects. As a result, they prepared a recombinant Factor C having the amino acid sequence of SEQ ID NO: 3 which can serve as, for example, an alternative to Factor C derived from *Limulus polyphemus* or a recombinant Factor C, thereby completing the present invention including such recombinant Factor C as an embodiment.
Further, the present inventors have conducted studies and found that thermostability of recombinant Factor C is surprisingly improved by introducing a given amino acid substitution into recombinant Factor C having the amino acid sequence of SEQ ID NO: 3, thereby completing the present invention including such finding as an embodiment.
The present disclosure include the following embodiments.

[1] A Factor C protein having 95% or higher amino acid sequence identity to SEQ ID NO: 3.
[2] A polynucleotide encoding the protein according to embodiment 1.
[3] A reagent for detecting endotoxin comprising the protein according to embodiment 1.
[4] A cell comprising the polynucleotide according to embodiment 2.
[5] A method for producing a Factor C protein comprising culturing the cell according to embodiment 4 and obtaining the Factor C protein according to embodiment 1.
[6] A method for producing a reagent for detecting endotoxin comprising culturing the cell according to embodiment 4 and obtaining the Factor C protein according to embodiment 1.
[7] A method for detecting endotoxin comprising a step of bringing the protein according to embodiment 1 or the reagent according to embodiment 3 into contact with a sample and a step of detecting endotoxin.

The present description encompasses the disclosed contents of Japanese Patent Application No. 2023-055723, which is the basis of the priority of the present application. Advantageous Effects of Invention
According to the present invention, a hydrolase can be obtained without relying on a limulus amebocyte lysate.

Brief Description of Drawings

[Figure 1] Figure 1 shows a schematic diagram demonstrating a principle of a method for endotoxin measurement using LAL.

Description of Embodiments

In an embodiment, the present disclosure provides a recombinant Factor C protein exhibiting 80% or higher, 85% or higher, 90% or higher, 91% or higher, 92% or higher, 93% or higher, 94% or higher, 95% or higher, 95.5% or higher, 96% or higher, 96.5% or higher, 97% or higher, 97.5% or higher, 98% or higher, 98.5% or higher, 99% or higher, or 99.5% or higher amino acid sequence identity to SEQ ID NO: 3.
In an embodiment, the present disclosure provides a recombinant Factor C protein comprising an amino acid sequence derived from the amino acid sequence of SEQ ID NO: 3 by substitution, deletion, or addition of one or several amino acids. The number of amino acids subjected to substitution, deletion, or addition indicated by the term "one or several" herein can be 1 to 20, for example, 1 to 19, 1 to 18, 1 to 17, 1 to 16, 1 to 15, 1 to 14, 1 to 13, 1 to 12, 1 to 11, 1 to 10, 1 to 9, 1 to 8, 1 to 7, 1 to 6, 1 to 5, 1 to 4, or 1 to 3, such as 1 or 2.

(Reference sequence)

In the present description, positions in a sequence are defined relative to SEQ ID NO: 3 as the reference sequence, unless specified otherwise. SEQ ID NO: 3 represents a novel recombinant Factor C protein provided by the present disclosure.

In the present description, "wild-type" refers to a trait present the most in nature in a conspecific group.

In the present description, a corresponding relationship of amino acid positions can readily be identified through comparison of amino acid sequences of various kinds of Factor C proteins using, for example, existing software for homology analysis of amino acids, such as GENETYX (manufactured by GENETYX CORPORATION). For example, the amino acid position of Factor C corresponding to position X in the amino acid sequence of SEQ ID NO: 3 can be identified by aligning the amino acid sequence of the Factor C with the amino acid sequence of SEQ ID NO: 3.

In an embodiment, a mutation can be artificially introduced into the recombinant Factor C represented by SEQ ID NO: 3. This can be achieved by artificially introducing the mutation in a sequence of a gene encoding recombinant Factor C.

The mutation may be artificially introduced with the intention of some specific effect or may be randomly or non-artificially introduced. Examples of mutations introduced with the intention of obtaining a specific effect include addition, deletion, or modification of the sequence to enhance the Factor C gene expression level, addition, deletion, or modification of the sequence to improve purification efficiency of the Factor C protein, and various kinds of mutations that confer a practically preferred property to the Factor C protein.

Amino acid sequence identity and gene sequence identity can be calculated by a program, such as maximum matching or search homology of GENETYX (manufactured by GENETYX), multiple alignment of CLUSTALW, or pairwise alignment of BLAST, herein. In order to calculate amino acid sequence identity, two or more Factor Cs may be aligned, and positions of identical amino acids in such two or more Factor Cs may be determined. Identical regions in amino acid sequences can be determined based on such information. The percent identity of two or more amino acid sequences is determined by subjecting two or more amino acid sequences to alignment using, for example, BLAST (BLASTP) targeting amino acid sequences by designating the total number of amino acids in the aligned region as the denominator and the number of identical amino acids relative to the total number as the numerator. As such, if no identity is found in parts of the two or more amino acid sequences, for example, an amino acid sequence comprises at its C terminus an additional sequence for which no identity can be observed, such regions cannot be aligned, and therefore, such regions are not used for calculation of the percent identity.

Further, positions of similar amino acids in two or more Factor Cs can be inspected. For example, a plurality of amino acid sequences can be subjected to alignment using CLUSTALW. In such case, Blosum62 is used as the algorithm and a plurality of amino acid sequences are subjected to alignment. Amino acids determined to be similar as a result of alignment may be referred to as "similar amino acids." In the mutant of the present disclosure, amino acid substitution can be carried out between such similar amino acids. Through such alignment, amino acid sequences composed of the identical amino acids or similar amino acids among a plurality of amino acid sequences can be investigated. Based on such information, homologous regions (also referred to as "highly conserved regions") in the amino acid sequences can be determined.

(High-throughput screening)

Factor C can further be subjected to high-throughput screening, so as to obtain a functional recombinant Factor C mutant. For example, a library of a transformant or transductant comprising the transgenic Factor C gene may be prepared and the resulting library may then be subjected to high-throughput screening using a microtiter plate. Alternatively, the library may be subjected to ultrahigh-throughput screening based on droplet microfluidics. For example, a combinatorial library of variant genes encoding variants can be constructed and a large population of variant Factor Cs can be subjected to screening by means of phage display (e.g., Chem. Rev., 105 (11): 4056-72, 2005), yeast display (e.g., Comb. Chem. High Throughput Screen., 2008; 11(2): 127-34), or bacterial display (e.g., Curr. Opin. Struct. Biol., 17: 474-80, 2007). Also see Agresti et al, "Ultrahigh-throughput screening in drop-based microfluidics for directed evolution," Proceedings of the National Academy of Sciences, 107 (9): 4004-4009, Mar, 2010. The description thereof concerning the technique for ultrahigh-throughput screening, which may be employed for screening of a Factor C variant, is incorporated herein by reference. For example, a library can be constructed by error-prone PCR. Alternatively, mutation may be introduced into a target, which is the region or position described herein or a region or position corresponding thereto, via saturation mutagenesis, so as to construct a library. Appropriate cells, such as electrocompetent EBY-100 cells, can be transformed using a library and approximately ten million types of mutants can be obtained. Yeast cells transformed with the library can then be subjected to cell sorting. A polydimethoxylsiloxane (PDMS) microfluidic device prepared via standard soft-lithography may be used. Monodisperse droplets can be prepared using a flow-focusing device. The prepared droplets separately comprising variants can be applied to an appropriate sorting device. Cells can be selected based on the presence or absence of Factor C activity. To this end, it is possible to use a reaction solution that becomes luminescent when, for example, the Factor C reacts therewith. For example, a 96-well plate, a 192-well plate, a 384-well plate, or a 9600-well plate and a plate reader may be used to measure luminescence. Alternatively, detection may be performed using a color-developing system or the fluorescent system instead of the luminescence system. Mutagenesis and selection may be repeated a plurality of rounds. The term "mutation" used herein encompasses substitution, insertion, deletion, and/or addition of amino acids.

For example, 1 to 10 mutations may be introduced into Factor C represented by SEQ ID NO: 3 to confirm the Factor C activity. Subsequently, 1 to 10 mutations can be further introduced into the recombinant Factor C mutant that is confirmed to have activity, and activity can then be confirmed. It is possible to repeat at least 2 rounds, 5 rounds, 10 rounds, 15 rounds, 20 rounds, 30 rounds, or 40 rounds, such as at least 50 rounds, of a series of high-throughput screening procedure (for example, the technique of obtaining and screening approximately ten million types of mutants described above). High-throughput screening introducing 1 or more or 5 or more mutations, such as 10 or more mutations, at each round may be repeated, for example, 10 times, and thus, it is possible to rapidly obtain a mutant derived from starting Factor C by introduction of 10 or more or 50 or more mutations, such as 100 or more mutations, and having activity of the starting Factor C. By repeating 20 rounds of the screening procedure, it is possible to rapidly obtain a mutant derived from a starting Factor C by introducing 20 or more or 100 or more mutations, such as 200 or more mutations, and having activity of the starting Factor C. The same applies to a procedure of 30 rounds, 40 rounds, or 50 rounds. Such procedure(s) can be performed using an automated apparatus by repeating a routine process.

It is possible to introduce a mutation into at least 1 position between the first amino acid and the last amino acid in the full-length amino acid sequence of Factor C. It should be noted that regions that are important for enzyme functions, such as the active center, substrate recognition site, endotoxin recognition site, and regions in the vicinity thereof, are excluded. Factor C is used in extensive fields of industry, and a person skilled in the art has a thorough knowledge on regions that are important for enzyme functions, including the active center, substrate recognition site, and endotoxin recognition site of the Factor C. In a particular embodiment, at the outset, one or a plurality of mutations can be introduced into, for example, positions 1 to 10 of the full-length Factor C sequence. Subsequently, one or a plurality of mutations can be introduced into positions 11 to 20 of the recombinant Factor C mutant that is confirmed to have activity, and activity of the resulting mutant can then be confirmed. This procedure can be repeated an "n" number of times ($n \leq 102$). For example, one or a plurality of mutations can be introduced into positions 1011 to 1019 on the 102$^{nd}$ instance. During the procedure, a region that is important for enzyme functions and a region that is not to be modified may be appropriately skipped. For example, position 966 of SEQ ID NO: 3 or a position equivalent thereto can be skipped. Thus, mutation of interest can be introduced into any position in the full-length sequence excluding regions that are important for enzyme function, and it is possible to rapidly obtain a recombinant Factor C mutant comprising a sequence derived from SEQ ID NO: 3 having, for example, 5 or more, 10 or more, 20 or more, 30 or more, 40 or more, 50 or more, 60 or more, 70 or more, 80 or more, 90 or more, 100 or more, 110 or more, 120 or more, 130 or more, 140 or more, 150 or more, 160 or more, 170 or more, 180 or more, or 190 or more, such as 200 or more mutations, and having activity. In an embodiment, mutation that would quench Factor C activity is not introduced. In an embodiment, mutations are not introduced into positions corresponding to positions 966, 809, 865, 26, 61, 62, and 63 of SEQ ID NO: 3. In an embodiment, positions 1 to 25 can be deleted from SEQ ID NO: 3.

A mutation may be introduced randomly or may be introduced by rational design. In an embodiment, the mutation to be introduced by rational design or mutation to be introduced randomly can be a conservative amino acid substitution. A conservative amino acid substitution includes an amino acid substitution in which an amino acid before substitution and an amino acid after substitution have similar chemical properties (e.g., Stryer et al., Biochemistry, Vol. 5, 2002, pp. 44 to 49). For example, conservative amino acid substitutions can be selected from the group consisting of: (i) substitution of a basic amino acid with a different type of a basic amino acid; (ii) substitution of an acidic amino acid with a different type of an acidic amino acid; (iii) substitution of an aromatic amino acid with a different type of an aromatic amino acid; (iv) substitution of a nonpolar aliphatic amino acid with a different type of a nonpolar aliphatic amino acid; and (v) substitution of a polar uncharged amino acid with a different type of a polar uncharged amino acid. A basic amino acid can be selected from among, for example, arginine, histidine, and lysine. An acidic amino acid can be, for example, aspartic acid or glutamic acid. An aromatic amino acid can be selected from among, for example, phenylalanine, tyrosine, and tryptophan. A nonpolar aliphatic amino acid can be selected from among, for example, glycine, alanine, valine, leucine, methionine, proline, and isoleucine. A polar uncharged amino acid can be selected from among, for example, serine, threonine, cysteine, asparagine, and glutamine. As a result of conservative amino acid substitution, an amino acid residue before substitution and an amino acid residue after substitution have similar chemical properties. In addition, positions subjected to conservative amino acid substitution exist at the same position in a protein tertiary structure, and, therefore, it is highly plausible that a variant having such conservative amino acid substitution maintains its tertiary structure and has activity.

In an embodiment, the mutation to be introduced by rational design or mutation to be introduced randomly includes a substitution with a functionally similar amino acids Tables of functionally similar amino acids are extensively known in the art. In an embodiment, for a substitution with a functionally similar amino acid, amino acids before substitution and amino acids after substitution can be between any of the amino acid classifications indicated below:

1) glycine (G), alanine (A);
2) aspartic acid (D), glutamic acid (E);
3) asparagine (N), glutamine (Q);
4) arginine (R), lysine (K), histidine (H);
5) isoleucine (I), leucine (L), valine (V), proline (P);
6) phenylalanine (F), tyrosine (Y), tryptophan (W);
7) serine (S), threonine (T); and
8) cysteine (C), methionine (M).

In contrast to conservative amino acid substitution, a nonconservative amino acid substitution of a given amino acid is performed with any amino acid that does not fall under the categories of conservative substitutions (i) to (v) outlined above. In an embodiment, amino acid substitution can be a nonconservative amino acid substitution. In such a case, it is possible to confirm as to, for example, whether or not protease activity before introduction of the nonconservative amino acid substitution would be maintained after introduction thereof. Such nonconservative amino acid substitution can be adopted if activity is confirmed.

In an embodiment, amino acid substitution can be substitution of an amino acid with a similar amino acid (similarity substitution). In the present specification, substitution of an amino acid with a similar amino acid is substitution of the amino acid with an amino acid that is evaluated to have positive or neutral (zero) scores by the amino acid substitution matrix used in the ClustalW software and the Blosum62 algorithm, unless specified otherwise (e.g., S. Heinkoff and J. G. Henikoff, Proc. Natl. Acad. Sci., U.S.A., Vol. 89, pp. 10915-10919, 1992, see, in particular, Figure 2 and Thompson, Nucleic Acid Research, 1994, Vol. 22, No. 22, pp. 4673-4680). The tables of matrices were prepared from about 2000 blocks of aligned sequence segments characterizing 500 or more groups of related proteins. In addition, substantially the same set of scores can be attained through repetitive application of a single matrix or a subset of protein groups. Thus, such substitution matrix is considered to be versatile. This is the most widely used approach utilizing the fact that homologous sequences are evolutionarily related to each other. Accordingly, it is highly plausible that a variant comprising similarity substitution introduced into a given Factor C has activity.

In an embodiment, a conservative amino acid substitution or substitution of an amino acid with a functionally similar amino acid is not present in regions that are important for enzyme functions, such as an active center, substrate recognition site, coenzyme recognition motif, and regions in the vicinity thereof, of an enzyme, and, therefore, such substitution will not significantly influence the enzyme activity. In another embodiment, conservative amino acid substitution or substitution with functionally similar amino acids is present in regions, such as an active center, substrate recognition site, coenzyme recognition motif, and regions in the vicinity thereof, of the enzyme, although such substitution does not substantially influence the enzyme activity.

A recombinant Factor C can have a deletion of an amino acid from the sequence before amino acid substitution. In a typical embodiment, there is no amino acid deletion in a region that is important for enzyme functions, and, accordingly, it would not significantly influence the enzyme activity. In an embodiment, the "deletion" can be a short deletion of 1 or 2 amino acids. When a given Factor C has an amino acid sequence that is different from the amino acid sequence of another Factor C by amino acid deletion, in an embodiment, such deletion can be introduced into the other Factor C. Since both Factor Cs have activity, it is highly likely that such deletion would not significantly influence the enzyme activity.

Recombinant Factor C can have insertion of additional amino acids in the sequence before mutation. In a typical embodiment, there is no amino acid insertion in a region that is important for enzyme functions, such as an active center, substrate recognition site, coenzyme recognition motif, and regions in the vicinity thereof, and therefore, it will not significantly influence the enzyme activity. In an embodiment, "insertion" can be insertion of 1 to 4 amino acids. When a given Factor C has an amino acid sequence that is different from the amino acid sequence of another Factor C by amino acid insertion, in an embodiment, such amino acid can be introduced into the other Factor C. Since both Factor Cs have activity, it is highly unlikely that such insertion would significantly influence the enzyme activity.

An example of amino acid insertion is provided. When SEQ ID NO: 3 is aligned with Factor C of another origin, for example, one amino acid (lysine) at position 433 only is present between position 432 and position 434 of SEQ ID NO: 3. In the Factor C of another origin, however, 2 amino acids are present at the corresponding position. As such, if one amino acid is inserted into a position between position 432 to position 434 of SEQ ID NO: 3, it is highly likely that such insertion will not significantly influence the enzyme activity.

In addition, a recombinant Factor C can have addition of additional amino acids to the sequence before mutation. In a typical embodiment, amino acid addition is performed at the N or C terminus of Factor C, and it would not significantly influence the enzyme activity. In an embodiment, the "addition" can be addition of 1 to 6 amino acids or 1 to 5 amino acids, such as 1 to 4 amino acids. An example of addition is, but is not limited to, a short stretch of histidine residues (e.g., 2 to 6 histidine residues) to assist Factor C purification. Another example of addition is, without limitation, addition of a signal peptide to assist Factor C expression. Examples of signal peptides include known signal sequences and sequences functionally equivalent thereto.

Mutation can be introduced into Factor C while refraining from destroying a secondary structure, such as an $\alpha$ helix structure or $\beta$ sheet structure, or structural motif. A region of a secondary structure can be identified using, for example, secondary structure prediction algorithms. An example of prediction algorithms is, but is not limited to, NetSurfP-2.0. The same applies to other structural motifs, such as nest or niche and the like. Factor C comprises, from the N terminus toward the C terminus, an EGF-like domain, 3 Sushi domains, an LCCL domain, a C-type lectin domain, 2 further Sushi domains, and a trypsin domain. Mutation may be introduced into Factor C while refraining from destroying these domains and higher-order structures.

An amino acid residue or an amino acid sequence motif that is essential for Factor C activity is not substituted, unless specified otherwise. In particular embodiments, although such position can be subjected to conservative amino acid substitution or similarity substitution, activity of the variant after substitution is to be confirmed. Unless specified otherwise, amino acid deletion or insertion is not performed at a position downstream or upstream of an amino acid residue that is essential for Factor C activity. A position downstream or upstream of an amino acid residue that is essential for Factor C activity is a position closer to the N or C terminus by 1 or 2 positions from the amino acid residue that is essential for Factor C activity. In a particular embodiment, although amino acid deletion or insertion can be performed at a position downstream or upstream of an amino acid residue that is essential for Factor C activity, in such case, activity of the variant after substitution is to be confirmed. Whether or not a variant has activity can be confirmed by a routine process, such as high-throughput screening.

Factor C has a catalytic triad consisting of serine, histidine, and aspartic acid (or glutamic acid). In SEQ ID NO: 3, serine at position 966, histidine at position 809, and aspartic acid at position 865 constitute the catalytic triad. In Factor C, an amino acid at the amino terminus is involved in endotoxin recognition. In SEQ ID NO: 3, arginine at position 1 is involved in endotoxin recognition. Factor C has an arginine-tryptophan-arginine motif (RWR), which is a motif recognizing endotoxin. In SEQ ID NO: 3, arginine at position 61, tryptophan at position 62, and arginine at position 63 constitute a motif recognizing endotoxin. In the present disclosure, mutations are not introduced into the positions indicated above, unless specified otherwise. In a particular embodiment, a mutation may be introduced into the positions indicated above although in such case, activity of the variant after mutation is to be confirmed. In a particular embodiment, an amino acid at the amino terminus may be lysine or arginine. Either one or both of arginine residues of the arginine-tryptophan-arginine motif (RWR) may be substituted with lysine, and tryptophan may be substituted with tyrosine or phenylalanine. A region from positions 1 to 25 of Factor C is removed from a mature protein, such region is considered to constitute a signal sequence, and the signal sequence is cleaved during the process of Factor C production. In SEQ ID NO: 3, a region from methionine at position 1 to serine at position 25 constitutes a signal sequence.

In an embodiment, the recombinant Factor C of the present disclosure has Factor C activity. The presence or absence of the Factor C activity can be determined, for example, in accordance with the method described in Examples.

(Polynucleotide)

In an embodiment, the present disclosure provides a polynucleotide encoding the recombinant Factor C (hereinafter also referred to as a "recombinant Factor C gene"). A sequence of a polynucleotide can readily be determined based on the amino acid sequence of the recombinant Factor C. An example of the polynucleotide encoding the amino acid sequence of SEQ ID NO: 3 is, but is not limited to, the polynucleotide of SEQ ID NO: 4.

In an embodiment, for example, a polynucleotide encoding recombinant Factor C can comprise:

(i) a nucleotide sequence exhibiting 85% or higher, 86% or higher, 87% or higher, 88% or higher, 89% or higher, and more preferably 90% or higher, 91% or higher, 92% or higher, 93% or higher, 94% or higher, 95% or higher, 96% or higher, 97% or higher, 98% or higher, or 99% or higher sequence identity to the nucleotide sequence of SEQ ID NO: 4 over the entire length and encoding active Factor C;
(ii) a nucleotide sequence derived from the nucleotide sequence of SEQ ID NO: 4 by substitution, deletion, or addition of one or several nucleotides and encoding active Factor C; or
(iii) a nucleotide sequence selected from the group consisting of SEQ ID NO: 4.

In order to obtain nucleotides encoding these Factor Cs, methods for cloning genes used in general can be applied. For example, chromosomal DNA or mRNA can be extracted from tissues or cells of a limulus having the Factor C producing ability by conventional methods. Further, cDNA can be synthesized using the mRNA as the template. The chromosomal DNA or cDNA thus obtained can be used to produce a library of the chromosomal DNA or the cDNA.

Subsequently, it is possible to obtain DNA comprising the full-length nucleotide encoding the target Factor C by a method of synthesizing an appropriate probe DNA based on the amino acid sequence of the Factor C and selecting the polynucleotide encoding the Factor C from the library of the chromosomal DNA or cDNA using the probe DNA prepared, or by preparing an appropriate primer DNA based on the amino acid sequence, amplifying DNA comprising the target nucleotide fragment encoding the Factor C by an appropriate polymerase chain reaction (PCR) method, such as a 5'-RACE method or a 3'-RACE method, and ligating these DNA fragments.

When the nucleotide sequence encoding the Factor C is already known, the nucleotide sequence may be artificially synthesized. Artificial gene synthesizing service is provided by, for example, Integrated DNA Technologies.

(Method for producing a Factor C gene)

The mutation treatment of the Factor C gene can be performed by any known method according to the intended mutation form. That is, a method that brings the Factor C gene or recombinant DNA, into which this gene is incorporated, into contact with an agent serving as a mutagen so as to allow the agent to act on the gene or DNA; an ultraviolet irradiation method; a genetic engineering method; a method making full use of protein engineering methods, or the like can be widely used.

Examples of the agent serving as the mutagen used for the mutation treatment above include hydroxylamine, N-methyl-N'-nitro-N-nitrosoguanidine, nitrous acid, sulfurous acid, hydrazine, formic acid, and 5-bromouracil.

Regarding various conditions of the contact and the reaction, conditions depending on the type of an agent to be used or the like can be employed, and the conditions are not particularly limited as long as a desired mutation can be actually induced in the Factor C gene. Usually, the contact and the reaction with the agent at a concentration of preferably 0.5 to 12 M, a reaction temperature of 20°C to 80°C for 10 minutes or more and preferably from 10 to 180 minutes allows for inducing the desired mutation. When ultraviolet irradiation is performed, it can be carried out in accordance with conventional methods as described above.

As a method making full use of protein engineering methods, in general, a method known as site-directed mutagenesis can be used.

In addition to the genetic modification method above, a desired modified Factor C gene can be directly synthesized using an organic synthesis method or an enzyme synthesis method.

The nucleotide sequence of the Factor C gene can be confirmed by, for example, a multi-capillary DNA analysis system, Applied Biosystems 3730xl DNA analyzer (manufactured by Thermo Fisher Scientific).

(Vector, host cell)

In an embodiment, the present disclosure relates to a vector comprising the polynucleotide. It is preferable for these Factor C genes to be ligated to various types of vectors in accordance with a conventional method for ease of handling. An example of a vector includes a plasmid, and in addition to such plasmid vector, any other vectors known to a person skilled in the art, such as a bacteriophage vector and a cosmid vector, can be used. The type of the vector can be selected depending on the host cell, and, specifically, pET16-b, pKK223-3, or the like can preferably be used.

In an embodiment, the present disclosure relates to a host cell comprising the polynucleotide or vector. The host cell, without limitation, it is a bacterium, such as *Escherichia coli* or *Bacillus subtilis,* a yeast cell, an insect cell, an animal cell (for example, a mammal cell), or a plant cell, and preferably is a bacterial cell, such as *Escherichia coli.*

(Transformation and transduction)

The Factor C gene obtained as described above can be incorporated into a vector, such as a bacteriophage, cosmid, or plasmid vector, used for transformation of a prokaryotic cell or an eukaryotic cell by conventional methods, and the transformation can be performed on the host corresponding to each vector by conventional methods. For example, a microorganism belonging to the genus *Escherichia,* such as the obtained recombinant DNA, may be used as the host, and transformation or transduction may be performed on, for example, the *Escherichia coli* K-12 strain or the *Escherichia coli* B strain, and preferably the *Escherichia coli* JM109 strain, the *Escherichia coli* DH5α strain, the *Escherichia coli* BL21 strain, the *Escherichia coli* BL21 (DE3) strain, and the like, to obtain relevant strains. As a method for transferring a recombinant vector into such host cell, for example, a method of recombinant DNA transfer can be performed in the presence of calcium ions when the host cell is a microorganism of *Escherichia coli.* Further a method of electroporation may be performed. The Factor C gene can be codon-optimized in accordance with the expression host cell.

(Method for producing recombinant Factor C)

In an embodiment, the present disclosure relates to a method for producing the recombinant Factor C comprising a step of culturing the host cell. Culturing can be performed by various kinds of known methods, and a method of solid culture can be employed, while a method of liquid culture is preferable.

The production method may comprise a step of culturing the host cell under a condition in which a recombinant Factor C protein can be expressed and optionally a step of isolating the recombinant Factor C from the culture product or culture fluid. Under the condition in which the recombinant Factor C protein can be expressed, a Factor C gene is transcribed and translated, and a polypeptide encoded by such gene is produced.

As the medium for culturing the host cells, for example, eukaryotic cells, such as yeast or fungal cells, a medium produced by adding one or more kinds of inorganic salts, such as sodium chloride, potassium dihydrogen phosphate, dipotassium hydrogenphosphate, magnesium sulfate, magnesium chloride, ferric chloride, ferric sulfate, or manganese sulfate, to one or more kinds of nitrogen sources, such as a yeast extract, triptone, peptone, a meat extract, corn steep liquor, or an exudate of soybean or wheat bran, and further adding a carbohydrate raw material, vitamin, or the like as necessary is used.

When mammalian cells are used as host cells, media that are commonly used for mammalian cell culture, such as DMEM medium (Sigma), RPMI 1640 medium (Sigma), and ExpiCHO™ Expression Medium (Thermo Fisher Scientific), can be used. Mammalian cell culture can be performed at, for example, 35°C to 38°C, such as 36°C to 38°C, in the presence of 5% to 8% $CO_2$, via static culture or suspension culture.

When insect cells are used as host cells, media that are commonly used for insect cell culture, such as Sf-900™ medium (Thermo Fisher), Sf-900™ II medium (Thermo Fisher), Sf-900™ III medium (Thermo Fisher), Gibco™ TC-100 insect medium (Thermo Fisher), Schneider's insect medium (Sigma-Aldrich), Grace's insect medium (Sigma-Aldrich), TNM-FH insect medium (Sigma-Aldrich), and Express Five™ SFM medium (Thermo Fisher), can be used. Examples of insect cells include, but are not limited to, cells that are commonly used for recombinant protein expression, such as IPLB-Sf9, IPLB-Sf21, IPLB-SF+, High-Five™, Schneider S2, and BmN. Known cell lines, such as cell lines derived from silkworms (*Bombyx mori*), cabbage armyworm (*Mamestra brassicae*), fall armyworm (*Spodoptera frugiperda*), cabbage looper (*Trichoplusia ni*), and common fruit fly (*Drosophila melanogaster*), cell lines derived from *Drosophila* germs, and cell lines derived from *Bombyx mori* germs, may be used.

Conditions for insect cell culture are not particularly limited, and conditions that are commonly applied for insect cell culture may be used. Alternatively, conditions that are commonly applied may be appropriately modified. Culturing can be performed at, for example, 25°C to 30°C, such as 26°C to 29°C or 27°C to 28°C, via shake culture or static culture.

A technique of protein expression with the use of insect cells as the host cell is not particularly limited, and a technique that is commonly applied for recombinant protein expression may be used. For example, insect cells may be infected with viruses comprising a gene encoding a target protein integrated therein. Examples of viruses used for inset cell transformation include known viruses such as *Baculovirus, Nucleopolyhedrovirus* (NPV), *Autographa californica* NPV (AcNPV), and *Bombix mori* NPV (BmNPV). Nucleic acids can be introduced into viruses in accordance with a conventional technique, such as homologous recombination using a transfer vector. Examples of transfer vectors include pPSC8 (Protein Sciences), pVL1393 (Pharmingen), and pFastBac (Invitrogen). Alternatively, a vector comprising a gene encoding a target protein integrated thereinto may be introduced into insect cells, and the gene may be integrated into the host chromosome, so as to express the recombinant protein. Examples of vectors include, but are not limited to, known insect vectors, such as the pAc series, the pVL series, the pIZ series, the pIZ/V5-His vector (Thermo Fisher), and pIZT/V5-His (Thermo Fisher).

When eukaryotic cells such as yeast or fungal cells are used as the host cell, it is appropriate to adjust the initial pH of the medium to 7 to 9. When eukaryotic cells such as yeast or fungal cells are used, culturing is preferably performed by aeration-agitation submerged culture, shake culture, static culture, or the like at a culture temperature of 20°C to 42°C, preferably at a culturing temperature of around 25°C to 37°C for 4 to 24 hours, and further preferably at a culturing temperature of around 25°C to 37°C for 8 to 16 hours. Examples of yeast include yeast belonging to the genera *Zygosaccharomyces, Saccharomyces, Pichia,* and *Candida.* Examples of fungi include fungi belonging to the genera *Aspergillus* and *Tricoderma.* Examples of plant cells include plant cells transformed with the use of *Agrobacterium.* Examples of mammalian cells include CHO and HEK293 cells.

After the completion of culturing, Factor C can be collected from the culture product by a conventional means of collecting enzymes. When eukaryotic cells such as yeast or fungal cells are used, for example, the enzyme of interest can be released from a microorganism body by performing ultrasonic disruption treatment, milling treatment, or the like on the microorganism body by conventional methods, extracting the present enzyme with lytic enzymes, such as lysozyme and the like, or shaking or allowing to stand still in the presence of toluene or the like for lysis. Then, this solution is, for example, filtrated or centrifuged to remove solid matter, nucleic acids are removed with streptomycin sulfate, protamine sulfate, manganese sulfate, or the like, according to need, ammonium sulfate, alcohol, acetone, or the like is added thereto, fractionation is performed, a precipitate is collected, and a crude enzyme of the Factor C can be then obtained. When using mammalian cells or insect cells, a culture liquid, culture supernatant, or a disrupted cell extract may be obtained by disrupting cells in accordance with a conventional technique or other means, nucleic acids may be removed according to need, or precipitation and the like, such as ammonium sulfate precipitation, may be performed, according to need. Thus, a crude enzyme of Factor C can be obtained.

In order to further obtain a purified enzyme of Factor C from the crude enzyme of the Factor C, for example, a gel filtration method using Sephadex, Superdex, Ultro-gel, or the like; an adsorption elution method using an ion exchange carrier, hydrophobic carrier, or hydroxyapatite; an electrophoresis method using polyacrylamide gel or the like; a sedimentation method, such as a sucrose density-gradient centrifugation and the like; an affinity chromatography method, or a fractionation method using a molecular sieving membrane, a hollow fiber membrane, or the like is appropriately selected and performed, or a combination of the same is performed, to obtain the purified Factor C enzyme. The desired Factor C can be thus obtained. Factor C may be subjected to sugar chain cleaving, or it may be used without sugar chain cleaving.

The Factor C produced can be used for a reagent for detecting endotoxin or a method for detecting endotoxin described herein. If the produced Factor C has relatively high thermostability, storage stability of such enzyme is relatively improved, and such enzyme can contribute to preparation of a detection reagent and a detection kit with availability and storage stability that are superior to those achieved with the use of a conventional Factor C.

(Reagent for detecting endotoxin)

In an embodiment, the present disclosure provides a reagent for detecting endotoxin containing recombinant Factor C. The reagent for detecting endotoxin can contain other components that are commonly contained in conventional reagents, such as a buffer, a stabilizer, and a synthetic substrate and the like, in addition to the recombinant Factor C. The form of a reagent is not particularly limited, and a reagent can be in the form of, for example, lyophilized powder or a liquid reagent and the like.

When a sample contains endotoxin, endotoxin converts Factor C into activated Factor C. The activated Factor C can hydrolyze a labeled peptide substrate. The hydrolyzed label can be detected by, for example, turbidimetry, the chromogenic substrate method, fluorescent substrate method, or luminescent substrate method. Endotoxin in the sample can thus be detected.

Endotoxin is not particularly limited, provided that it is recognized as a substrate by the Factor C being used, and it may be naturally-occurring or chemically synthesized endotoxin. Any known endotoxin derivative can also be used.

In an embodiment, the present disclosure provides a kit for detecting endotoxin. The kit for detecting endotoxin can contain a reagent for detecting endotoxin and, according to need, instructions, a reference substance, a syringe, a needle, an equipment for sampling from an analyte, and the like.

(Method for detecting endotoxin)

In an embodiment, the present disclosure relates to a method for detecting endotoxin comprising using recombinant Factor C. The method can comprise a step of bringing a sample into contact with recombinant Factor C and a step of detecting a labeled product generated from a synthetic substrate.

In an embodiment, the recombinant Factor C having the sequence of SEQ ID NO: 3 or a recombinant Factor C protein exhibiting 80% or higher, 85% or higher, 90% or higher, 91% or higher, 92% or higher, 93% or higher, 94% or higher, 95% or higher, 95.5% or higher, 96% or higher, 96.5% or higher, 97% or higher, 97.5% or higher, 98% or higher, 98.5% or higher, 99% or higher, or 99.5% or higher amino acid sequence identity to SEQ ID NO: 3 can have a further amino acid substitution. Examples of positions that can be subjected to amino acid substitution include, but are not limited to, positions corresponding to the positions 423, 557, 669, 970, 973, 425, 605, 832, 833, 842, 876, 897, 914, 930, 955, 977, and 1013 of SEQ ID NO: 3. A recombinant Factor C mutant having one or more of such amino acid substitutions can have 79% or higher, 80% or higher, 85% or higher, 90% or higher, 91% or higher, 92% or higher, 93% or higher, 94% or higher, 95% or higher, 95.5% or higher, 96% or higher, 96.5% or higher, 97% or higher, 97.5% or higher, 98% or higher, 98.5% or higher, 99% or higher, or 99.5% or higher amino acid sequence identity to SEQ ID NO: 3.

In an embodiment, the recombinant Factor C can have an amino acid substitution indicated below, although the substituted amino acid is not limited thereto. A mutant comprising any one of the mutations indicated below may be referred to as the "single mutant Mon" for the convenience of description.

[Table 1]

| Site of substitution (Reference sequence: SEQ ID NO: 3) | Amino acid after substitution |
|---|---|
| F423 | L |
| F557 | L |

(continued)

| Site of substitution (Reference sequence: SEQ ID NO: 3) | Amino acid after substitution |
|---|---|
| F669 | Y |
| L970 | A, V |
| S973 | V, I, L |
| Y425 | F |
| Y605 | F |
| Y832 | F |
| Y833 | F |
| Y842 | K |
| A876 | R |
| K897 | Q |
| T914 | R |
| S930 | D, E, K |
| K955 | E |
| R977 | E |
| S1013 | D |

(Multiple mutant)

In an embodiment, the recombinant Factor C may comprise 2 or more, 3 or more, 4 or more, 5 or more, 6 or more, 7 or more, 8 or more, 9 or more, 10 or more, 11 or more, 12 or more, 13 or more, 14 or more, 15 or more, or 16 or more, such as 17, of the amino acid substitutions indicated above.

In an embodiment, the recombinant Factor C may comprise the amino acid substitutions indicated below. Parenthesis means either of the amino acids indicated therein may be used. For example, "L973(V,I,L)" indicates that a position corresponding to position 973 of SEQ ID NO: 3 may be Val, Ile, or Leu.

[Double mutant Dou]
L970(A,V)/F557(L), L970(A,V)/F669(Y), L970(A,V)/S973(V,I,L), L970(A,V)/W832(F), L970(A,V)/K897Q, L970(A,V)/S930(D,E,K), F557(L)/F669(Y), F557(L)/S973(V,I,L), F557(L)/W832(F), F557(L)/K897Q, F557(L)/S930(D,E,K), F669(Y)/S973(V,I,L), F669(Y)/W832(F), F669(Y)/K897Q, F669(Y)/S930(D,E,K), S973(V,I,L)/W832(F), S973(V,I,L)/K897Q, S973(V,I,L)/S930(D,E,K), W832(F)/K897Q, W832(F)/S930(D,E,K), and K897Q/S930(D,E,K).

[Triple mutant Tri]
L970(A,V)/F557(L)/F669(Y), L970(A,V)/F557(L)/S973(V,I,L), L970(A,V)/F557(L)/W832(F), L970(A,V)/F557(L)/K897Q, L970(A,V)/F557(L)/S930(D,E,K), F557(L)/F669(Y)/S973(V,I,L), F557(L)/F669(Y)/W832(F), F557(L)/F669(Y)/K897Q, F557(L)/F669(Y)/S930(D,E,K), F669(Y)/S973(V,I,L)/W832(F), F669(Y)/S973(V,I,L)/K897Q, F669(Y)/S973(V,I,L)/S930(D,E,K), S973(V,I,L)/W832(F)/K897Q, S973(V,I,L)/W832(F)/S930(D,E,K), and W832(F)/K897Q/S930(D,E,K).

[Quadruple mutant Qua]
L970(A,V)/F557(L)/F669(Y)/S973(V,I,L), L970(A,V)/F557(L)/F669(Y)/W832(F), L970(A,V)/F557(L)/-F669(Y)/K897Q, L970(A,V)/F557(L)/F669(Y)/S930(D,E,K), F557(L)/F669(Y)/S973(V,I,L)/W832(F), F557(L)/F669(Y)/S973(V,I,L)/K897Q, F557(L)/F669(Y)/S973(V,I,L)/S930(D,E,K), F669(Y)/S973(V,I,L)/W832(F)/K897Q, F669(Y)/S973(V,I,L)/W832(F)/S930(D,E,K), and S973(V,I,L)/W832(F)/K897Q/S930(D,E,K).

[Quintuple mutant Pen]
L970(A,V)/F557(L)/F669(Y)/S973(V,I,L)/W832(F), L970(A,V)/F557(L)/F669(Y)/S973(V,I,L)/K897Q, L970(A,V)/F557(L)/F669(Y)/S973(V,I,L)/S930(D,E,K), L970(A,V)/F557(L)/F669(Y)/S973(V,I,L)/Y425(F), L970(A,V)/F557(L)/F669(Y)/S973(V,I,L)/Y605(F), L970(A,V)/F557(L)/F669(Y)/S973(V,I,L)/Y833(F), L970(A,V)/F557(L)/F669(Y)/S973(V,I,L)/Y842(K), L970(A,V)/F557(L)/F669(Y)/S973(V,I,L)/A876(R), L970(A,V)/F557(L)/F669(Y)/S973(V,I,L)/T914(R), L970(A,V)/F557(L)/F669(Y)/S973(V,I,L)/K955(E), L970(A,V)/F557(L)/F669(Y)/S973(V,I,L)/R977(E), L970(A,V)/F557(L)/F669(Y)/5973(V,I,L)/S1013(D), F557(L)/F669(Y)/S973(V,I,L)/W832(F)/K897Q, F557(L)/F669(Y)/S973(V,I,L)/W832(F)/S930(D,E,K), and F669(Y)/S973(V,I,L)/W832(F)/K897Q/S930(D,E,K).

[Sextuple mutant Hex]

L970(A,V)/F557(L)/F669(Y)/S973(V,I,L)/W832(F)/K897Q, L970(A,V)/F557(L)/-F669(Y)/S973(V,I,L)/W832(F)/S930(D,E,K), L970(A,V)/F557(L)/F669(Y)/S973(V,I,L)/W832(F)/A876(R), L970(A,V)/F557(L)/F669(Y)/S973(V,I,L)/W832(F)/Y842(K), L970(A,V)/F557(L)/F669(Y)/S973(V,I,L)/K897-Q/A876(R), L970(A,V)/F557(L)/F669(Y)/S973(V,I,L)/K897Q/Y842(K), L970(A,V)/F557(L)/-F669(Y)/S973(V,I,L)/S930(D,E,K)/A876(R), L970(A,V)/F557(L)/F669(Y)/S973(V,I,L)/Y842(K)/Y425(F), L970(A,V)/F557(L)/F669(Y)/S973(V,I,L)/Y842(K)/Y605(F), L970(A,V)/F557(L)/-F669(Y)/S973(V,I,L)/Y842(K)/Y833(F), L970(A,V)/F557(L)/F669(Y)/S973(V,I,L)/Y842(K)/Y842(K), L970(A,V)/F557(L)/F669(Y)/S973(V,I,L)/Y842(K)/A876(R), L970(A,V)/F557(L)/-F669(Y)/S973(V,I,L)/Y842(K)/T914(R), L970(A,V)/F557(L)/F669(Y)/S973(V,I,L)/Y842(K)/K955(E), L970(A,V)/F557(L)/F669(Y)/S973(V,I,L)/Y842(K)/R977(E), L970(A,V)/F557(L)/-F669(Y)/S973(V,I,L)/Y842(K)/S1013(D), F557(L)/F669(Y)/S973(V,I,L)/W832(F)/K897Q/S930(D,E,K), F557(LYF669(Y)/S973(V,I,L)Y/W832(F)/K897Q/A876(R), F557(L)/F669(Y)/S973(V,I,L)/W832(F)/K897Q/K897Q, F557(L)/F669(Y)/S973(V,I,L)/W832(F)/K897Q/S930(D,E,K), F557(L)/F669(Y)/S973(V,I,L)/W832(F)/K897Q/K955(E), F557(L)/F669(Y)/S973(V,I,L)/W832(F)/K897Q/R977(E), and F557(L)/F669(Y)/S973(V,I,L)/W832(F)/K897Q/S1013(D).

[Septuple mutant Hep]

L970(A,V)/F557(L)/F669(Y)/S973(V,I,L)/W832(F)/K897Q/S930(D,E,K), L970(A,V)/F557(L)/-F669(Y)/S973(V,I,L)/Y842(K)/A876(R)/R977(E), L970(A,V)/F557(L)/-F669(Y)/S973(V,I,L)/Y842(K)/A876(R)/S1013(D), L970(A,V)/F557(L)/-F669(Y)/S973(V,I,L)/Y842(K)/A876(R)/K897Q, L970(A,V)/F557(L)/-F669(Y)/S973(V,I,L)/Y842(K)/A876(R)/S930(D,E,K), L970(A,V)/F557(L)/-F669(Y)/S973(V,I,L)/Y842(K)/A876(R)/K955(E), L970(A,V)/F557(L)/-F669(Y)/S973(V,I,L)/Y842(K)/A876(R)/W832(F), L970(A,V)/F557(L)/F669(Y)/S973(V,I,L)/Y842(K)/A876(R)/-Y425(F), L970(A,V)/F557(L)/F669(Y)/S973(V,I,L)/Y842(K)/A876(R)/Y605(F), L970(A,V)/F557(L)/-F669(Y)/S973(V,I,L)/Y842(K)/A876(R)/Y833(F), L970(A,V)/F557(L)/-F669(Y)/S973(V,I,L)/Y842(K)/A876(R)/T914(R), L970(A,V)/F557(L)/-F669(Y)/S973(V,I,L)/Y842(K)/A876(R)/R977(E), L970(A,V)/F557(L)/-F669(Y)/S973(V,I,L)/Y842(K)/A876(R)/S1013(D), F557(L)/F669(Y)/S973(V,I,L)/W832(F)/K897Q/S930(D,E,K)/R977(E), F557(L)/F669(Y)/S973(V,I,L)/W832(F)/K897Q/S930(D,E,K)/S1013(D), F557(L)/F669(Y)/S973(V,I,L)/W832(F)/K897Q/S930(D,E,K)/K955(E), F557(L)/F669(Y)/S973(V,I,L)/W832(F)/K897Q/S930(D,E,K)/Y425(F), F557(L)/F669(Y)/S973(V,I,L)/W832(F)/K897Q/S930(D,E,K)/Y605(F), F557(L)/F669(Y)/S973(V,I,L)/W832(F)/K897Q/S930(D,E,K)/Y833(F), F557(L)/F669(Y)/S973(V,I,L)/W832(F)/K897Q/S930(D,E,K)/T914(R), and F557(L)/F669(Y)/S973(V,I,L)/W832(F)/K897Q/S930(D,E,K)/R977(E).

[Octuple mutant Oct]

L970(A,V)/F557(L)/F669(Y)/S973(V,I,L)/Y842(K)/A876(R)/R977(E)/S1013(D), L970(A,V)/F557(L)/-F669(Y)/S973(V,I,L)/Y842(K)/A876(R)/R977(E)/K897Q, L970(A,V)/F557(L)/-F669(Y)/S973(V,I,L)/Y842(K)/A876(R)/R977(E)/S930(D,E,K), L970(A,V)/F557(L)/-F669(Y)/S973(V,I,L)/Y842(K)/A876(R)/R977(E)/K955(E), L970(A,V)/F557(L)/-F669(Y)/S973(V,I,L)/Y842(K)/A876(R)/R977(E)/W832(F), L970(A,V)/F557(L)/-F669(Y)/S973(V,I,L)/Y842(K)/A876(R)/R977(E)/Y425(F), L970(A,V)/F557(L)/-F669(Y)/S973(V,I,L)/Y842(K)/A876(R)/R977(E)/Y605(F), L970(A,V)/F557(L)/-F669(Y)/S973(V,I,L)/Y842(K)/A876(R)/R977(E)/Y833(F), L970(A,V)/F557(L)/-F669(Y)/S973(V,I,L)/Y842(K)/A876(R)/R977(E)/T914(R), L970(A,V)/F557(L)/-F669(Y)/S973(V,I,L)/Y842(K)/A876(R)/S1013(D)/K897Q, L970(A,V)/F557(L)/-F669(Y)/S973(V,I,L)/Y842(K)/A876(R)/S1013(D)/S930(D,E,K), L970(A,V)/F557(L)/-F669(Y)/S973(V,I,L)/Y842(K)/A876(R)/S1013(D)/K955(E), L970(A,V)/F557(L)/-F669(Y)/S973(V,I,L)/Y842(K)/A876(R)/S1013(D)/W832(F), L970(A,V)/F557(L)/-F669(Y)/S973(V,I,L)/Y842(K)/A876(R)/S1013(D)/Y425(F), L970(A,V)/F557(L)/-F669(Y)/S973(V,I,L)/Y842(K)/A876(R)/S1013(D)/Y605(F), L970(A,V)/F557(L)/-F669(Y)/S973(V,I,L)/Y842(K)/A876(R)/S1013(D)/Y833(F), L970(A,V)/F557(L)/-F669(Y)/S973(V,I,L)/Y842(K)/A876(R)/S 10 1 3(D)/T914(R), F557(L)/F669(Y)/S973(V,I,L)/Y842(K)/A876(R)/R977(E)/S1013(D)/K897Q,
F557(L)/F669(Y)/S973(V,I,L)/Y842(K)/A876(R)/R977(E)/S1013(D)/S930(D,E,K),                and
F557(L)/F669(Y)/S973(V,I,L)/Y842(K)/A876(R)/R977(E)/S1013(D)/K955(E).
[Nonuple mutant Non]

L970(A,V)/F557(L)/F669(Y)/S973(V,I,L)/Y842(K)/A876(R)/R977(E)/S1013(D)/K897Q,        L970(A,V)/F557(L)/-
F669(Y)/S973(V,I,L)/Y842(K)/A876(R)/R977(E)/S1013(D)/S930(D,E, K),
L970(A,V)/F557(L)/F669(Y)/S973(V,I,L)/Y842(K)/A876(R)/R977(E)/S1013(D)/K955(E),        L970(A,V)/F557(L)/-
F669(Y)/S973(V,I,L)/Y842(K)/A876(R)/R977(E)/S1013(D)/W832(F),        L970(A,V)/F557(L)/-
F669(Y)/S973(V,I,L)/Y842(K)/A876(R)/R977(E)/S1013(D)/Y425(F),        L970(A,V)/F557(L)/-
F669(Y)/S973(V,I,L)/Y842(K)/A876(R)/R977(E)/S1013(D)/Y605(F),        L970(A,V)/F557(L)/-
F669(Y)/S973(V,I,L)/Y842(K)/A876(R)/R977(E)/S1013(D)/Y833(F),        L970(A,V)/F557(L)/-
F669(Y)/S973(V,I,L)/Y842(K)/A876(R)/R977(E)/S1013(D)/        T914(R),
FS57(L)/F669(Y)/5973(V,I,L)/Y842(K)/A876(R)/R977(E)/S1013(D)/K897Q/5930(D,E,K),
F557(L)/F669(Y)/S973(V,I,L)/Y842(K)/A876(R)/R977(E)/S1013(D)/K897Q/K955(E),        and
F557(L)/F669(Y)/S973(V,I,L)/Y842(K)/A876(R)/R977(E)/S1013(D)/S930(D,E,K)/K955(E).

The group of mutants indicated above is referred to as Group S-1 for the convenience of description.

[Equation 1]

$$S\text{-}1 = \{Mon, Dou, Tri, Qua, Pen, Hex, Hep, Oct, Non\}$$

In an embodiment, the recombinant Factor C can have the amino acid substitutions indicated below. Parenthesis means either of the amino acids indicated therein may be used.

[Single mutant Mon-2]
F423(L), Y425(F), Y605(F), Y833(F), Y842(K), A876(R), T914(R), K955(E), R977(E), and S1013(D).
[Double mutant Dou-2]
F423(L)/Y425(F), F423(L)/Y833(F), F423(L)/Y842(K), F423(L)/A876(R), F423(L)/T914(R), F423(L)/K955(E),
F423(L)/R977(E), F423(L)/S1013(D), Y425(F)/Y833(F), Y425(F)/Y842(K), Y425(F)/A876(R), Y425(F)/T914(R),
Y425(F)/K955(E), Y425(F)/R977(E), Y425(F)/S1013(D), Y833(F)/Y842(K), Y833(F)/A876(R), Y833(F)/T914(R),
Y833(F)/K955(E), Y833(F)/R977(E), Y833(F)/S1013(D), Y842(K)/A876(R), Y842(K)/T914(R), Y842(K)/K955(E),
Y842(K)/R977(E), Y842(K)/S1013(D), A876(R)/T914(R), A876(R)/K955(E), A876(R)/R977(E),
A876(R)/S1013(D), T914(R)/K955(E), T914(R)/R977(E), T914(R)/S1013(D), K955(E)/R977(E),
K955(E)/S1013(D), and R977(E)/S1013(D).
[Triple mutant Tri-2]
F423(L)/Y425(F)/Y833(F), F423(L)/Y425(F)/Y842(K), F423(L)/Y425(F)/A876(R), F423(L)/Y425(F)/T914(R),
F423(L)/Y425(F)/K955(E), F423(L)/Y425(F)/R977(E), F423(L)/Y425(F)/S1013(D), Y425(F)/Y833(F)/Y842(K),
Y425(F)/Y833(F)/A876(R), Y425(F)/Y833(F)/T914(R), Y425(F)/Y833(F)/K955(E), Y425(F)/Y833(F)/R977(E),
Y425(F)/Y833(F)/S1013(D), Y833(F)/Y842(K)/A876(R), Y833(F)/Y842(K)/T914(R), Y833(F)/Y842(K)/K955(E),
Y833(F)/Y842(K)/R977(E), Y833(F)/Y842(K)/S1013(D), Y842(K)/A876(R)/T914(R), Y842(K)/A876(R)/K955(E),
Y842(K)/A876(R)/R977(E), Y842(K)/A876(R)/S1013(D), A876(R)/T914(R)/K955(E), A876(R)/T914(R)/R977(E),
A876(R)/T914(R)/S1013(D), T914(R)/K955(E)/R977(E), T914(R)/K955(E)/S1013(D), and
K955(E)/R977(E)/S1013(D).
[Quadruple mutant Qua-2]
F423(L)/Y425(F)/Y833(F)/Y842(K), F423(L)/Y425(F)/Y833(F)/A876(R), F423(L)/Y425(F)/Y833(F)/T914(R),
F423(L)/Y425(F)/Y833(F)/K955(E), F423(L)/Y425(F)/Y833(F)/R977(E), F423(L)/Y425(F)/Y833(F)/S1013(D),
Y425(F)/Y833(F)/Y842(K)/A876(R), Y425(F)/Y833(F)/Y842(K)/T914(R), Y425(F)/Y833(F)/Y842(K)/K955(E),
Y425(F)/Y833(F)/Y842(K)/R977(E), Y425(F)/Y833(F)/Y842(K)/S1013(D), Y833(F)/Y842(K)/A876(R)/T914(R),
Y833(F)/Y842(K)/A876(R)/K955(E), Y833(F)/Y842(K)/A876(R)/R977(E), Y833(F)/Y842(K)/A876(R)/S1013(D),
Y842(K)/A876(R)/T914(R)/K955(E), Y842(K)/A876(R)/T914(R)/R977(E), Y842(K)/A876(R)/T914(R)/S1013(D),
A876(R)/T914(R)/K955(E)/R977(E), A876(R)/T914(R)/K955(E)/S1013(D), and
T914(R)/K955(E)/R977(E)/S1013(D).
[Quintuple mutant Pen-2]
F423(L)/Y425(F)/Y833(F)/Y842(K)/A876(R),        F423(L)/Y425(F)/Y833(F)/Y842(K)/T914(R),
F423(L)/Y425(F)/Y833(F)/Y842(K)/K955(E),        F423(L)/Y425(F)/Y833(F)/Y842(K)/R977(E),
F423(L)/Y425(F)/Y833(F)/Y842(K)/S1013(D),        Y425(F)/Y833(F)/Y842(K)/A876(R)/T914(R), Y425(F)/Y833(F)/Y842(K)/A876(R)/K955(E), Y425(F)/Y833(F)/Y842(K)/A876(R)/R977(E),
Y425(F)/Y833(F)/Y842(K)/A876(R)/S1013(D), Y833(F)/Y842(K)/A876(R)/T914(R)/K955(E),
Y833(F)/Y842(K)/A876(R)/T914(R)/R977(E), Y833(F)/Y842(K)/A876(R)/T914(R)/S1013(D),
Y842(K)/A876(R)/T914(R)/K955(E)/R977(E), Y842(K)/A876(R)/T914(R)/K955(E)/S1013(D), and
A876(R)/T914(R)/K955(E)/R977(E)/S1013(D).
[Sextuple mutant Hex-2]
F423(L)/Y425(F)/Y833(F)/Y842(K)/A876(R)/T914(R), F423(L)/Y425(F)/Y833(F)/Y842(K)/A876(R)/K955(E),
F423(L)/Y425(F)/Y833(F)/Y842(K)/A876(R)/R977(E), F423(L)/Y425(F)/Y833(F)/Y842(K)/A876(R)/S1013(D),
Y425(F)/Y833(F)/Y842(K)/A876(R)/T914(R)/K955(E), Y425(F)/Y833(F)/Y842(K)/A876(R)/T914(R)/R977(E),
Y425(F)/Y833(F)/Y842(K)/A876(R)/T914(R)/S1013(D), Y833(F)/Y842(K)/A876(R)/T914(R)/K955(E)/R977(E),
Y833(F)/Y842(K)/A876(R)/T914(R)/K955(E)/S1013(D), and Y842(K)/A876(R)/T914(R)/-
K955(E)/R977(E)/S1013(D).
[Septuple mutant Hep-2]
F423(L)/Y425(F)/Y833(F)/Y842(K)/A876(R)/T914(R)/K955(E),
F423(L)/Y425(F)/Y833(F)/Y842(K)/A876(R)/T914(R)/R977(E),
F423(L)/Y425(F)/Y833(F)/Y842(K)/A876(R)/T914(R)/S1013(D), Y425(F)/Y833(F)/Y842(K)/A876(R)/T914(R)/-
K955(E)/R977(E), Y425(F)/Y833(F)/Y842(K)/A876(R)/T914(R)/K955(E)/S1013(D), and
Y833(F)/Y842(K)/A876(R)/T914(R)/K955(E)/R977(E)/S1013(D).
[Octuple mutant Oct-2]
F423(L)/Y425(F)/Y833(F)/Y842(K)/A876(R)/T914(R)/K955(E)/R977(E),
F423(L)/Y425(F)/Y833(F)/Y842(K)/A876(R)/T914(R)/K955(E)/S1013(D), and
Y425(F)/Y833(F)/Y842(K)/A876(R)/T914(R)/K955(E)/R977(E)/S1013(D).
[Nonuple mutant Non-2]
F423(L)/Y425(F)/Y833(F)/Y842(K)/A876(R)/T914(R)/K955(E)/R977(E)/S1013(D).

The group of mutants indicated above is referred to as "Group S-2" for the convenience of description.

[Equation 2]

$$S\text{-}2 = \{\text{Mon-2, Dou-2, Tri-2, Qua-2, Pen-2, Hex-2, Hep-2, Oct-2, Non-2}\}$$

In an embodiment, the recombinant Factor C may comprise mutations of Group S-1 and of Group S-2 in any combination. A group of such mutants is referred to as "Group S-3" for the convenience of description. In an embodiment, the recombinant Factor C mutant may comprise at least 1 reversion starting from the recombinant Factor C mutant included in Group S-1, S-2, or S-3 (however, the number of reversion does not exceed the number of amino acid substitutions introduced). The term "reversion" indicates a mutation whereby a mutation is introduced into a site to which a given amino acid substitution has been introduced to revert the amino acid to the amino acid before the amino acid substitution. A group of such revertants is referred to as "Group S-4" for the convenience of description.

The recombinant Factor C mutant included in Group S-1, S-2, S-3, or S-4 can have thermostability improved compared to that of the recombinant Factor C before mutation. Such mutant may be referred to as "a mutant with improved thermostability" herein. Recombinant Factor C mutants included in Group S-1, S-2, S-3, or S-4 are finite, and a person skilled in the art can verify the thermostability thereof by a routine process. In an embodiment, the present disclosure provides a recombinant Factor C protein mutant having mutation included in Group S-1, S-2, S-3, or S-4 and exhibiting 80% or higher, 85% or higher, 90% or higher, 91% or higher, 92% or higher, 93% or higher, 94% or higher, 95% or higher, 95.5% or higher, 96% or higher, 96.5% or higher, 97% or higher, 97.5% or higher, 98% or higher, 98.5% or higher, 99% or higher, or 99.5% or higher amino acid sequence identity to SEQ ID NO: 3.

In the present description, "thermostability" can be evaluated by using, for example, the residual activity measured upon heat treatment of Factor C at a predetermined temperature for a predetermined period of time as an indicator. More specifically, the thermostability of the Factor C can be evaluated by comparing residual activity rates of the Factor Cs after heat treatment under high temperature conditions, for example, at a temperature from 30°C to 56°C, such as 35°C to 55°C, 40°C to 50°C, 42°C to 49°C, or 45°C to 49°C for a certain period of time, for example, from 5 to 120 minutes or from 10 to 60 minutes, such as from 15 to 30 minutes.

The residual activity of a Factor C is the activity after the heat treatment relative to the Factor C activity before applying the above-described high temperature condition, which is designated to be 1. For example, when the residual activity after the heat treatment is halved, for example, the residual activity after the heat treatment is 0.5 relative to the activity before the heat treatment, which is designated to be 1. Residual activity of the Factor C after heat treatment is calculated relative to the Factor C activity before applying the high temperature condition describes above. In the present specification, improvement of thermostability refers to the case where the residual activity when the Factor C mutant is subjected to the above-identified conditions is improved by at least 1.01 times, 1.02 times, 1.05 times, 1.1 times, 1.2 times, 1.3 times, 1.4 times, 1.5 times, 1.6 times, 1.7 times, 1.8 times, 1.9 times, or 2 times, relative to the residual activity of the Factor C before introduction of the mutations of the present disclosure (i.e., the mutations included in Group S-1, S-2, S-3, or S-4). In other words, in the present specification, improvement of thermostability refers to the case where the residual activity when the recombinant Factor C is subjected to the reaction under the above-identified conditions is improved by at least 1%, 2%, 5%, 10%, 20%, 30%, 40%, 50%, 60%, 70%, 80%, 90%, or 100%, relative to the residual activity of the Factor C before introduction of the mutations of the present disclosure.

In an embodiment, Factor C may comprise an amino acid sequence selected from the group consisting of (i) to (iii) below:

(i) an amino acid sequence exhibiting 80% or higher, 85% or higher, 90% or higher, 91% or higher, 92% or higher, 93% or higher, 94% or higher, 95% or higher, 95.5% or higher, 96% or higher, 96.5% or higher, 97% or higher, 97.5%, 98% or higher, 98.5%, 99% or higher, or 99.5% or higher amino acid sequence identity to SEQ ID NO: 3;

(ii) an amino acid sequence of SEQ ID NO: 3; and

(iii) an amino acid sequence having at least 1 mutation included in Group S-1, S-2, S-3, or S-4 in the amino acid sequence (i) or (ii), such as substitution of an amino acid to Leu at a position corresponding to F423, substitution of an amino acid to Leu at a position corresponding to F557, substitution of an amino acid to Tyr at a position corresponding to F669, substitution of an amino acid to Ala or Val at a position corresponding to L970, substitution of an amino acid to Val, Ile, or Leu at a position corresponding to S973, substitution of an amino acid to Phe at a position corresponding to Y425, substitution of an amino acid to Phe at a position corresponding to Y605, substitution of an amino acid to Phe at a position corresponding to Y832, substitution of an amino acid to Phe at a position corresponding to Y833, substitution of an amino acid to Lys at a position corresponding to Y842, substitution of an amino acid to Arg at a position corresponding to A876, substitution of an amino acid to Gln at a position corresponding to K897, substitution of an amino acid to Arg at a position corresponding to T914, substitution of an amino acid to Asp, Glu, or Lys at a position corresponding to S930, substitution of an amino acid to Glu at a position corresponding to K955, substitution of an amino acid to Glu at a position corresponding to R977, and/or substitution of an amino acid to Asp at a position corresponding to S1013 of SEQ ID NO: 3.

In an embodiment, the present disclosure provides a Factor C having residual activity after heat treatment at 45°C for 30 minutes, which is higher than 67% relative to the activity before the heat treatment (100%), such as 68% or higher, 75% or higher, 80% or higher, 83% or higher, 85% or higher, 90% or higher, 92% or higher, 95% or higher, or 99% or higher. In an embodiment, the present disclosure provides Factor C having residual activity after heat treatment at 48°C for 30 minutes, which is higher than 3% relative to the activity before the heat treatment (100%), such as 50% or higher, 77% or higher, 79% or higher, 81% or higher, 82% or higher, 83% or higher, 84% or higher, 90% or higher, 92% or higher, 97% or higher, 99% or higher, 102% or higher, 104% or higher, or 113% or higher. In an embodiment, the present disclosure provides Factor C having residual activity after heat treatment at 49°C for 30 minutes, which is higher than 62% relative to the activity before the heat treatment (100%), such as 63% or higher, 70% or higher, 76% or higher, 77% or higher, 81% or higher, 94% or higher, 103% or higher, or 121% or higher.

Wild-type sequences are excluded from the scope of the recombinant Factor C of the present disclosure. Further, naturally-occurring Factor Cs are excluded from the scope of the recombinant Factor C of the present disclosure. In an embodiment, wild-type sequences and naturally-occurring Factor C proteins that were found after filing or publication of the present disclosure are also excluded.

According to the present disclosure, a hydrolase can be obtained without relying on a limulus amebocyte lysate. Further, according to the present disclosure, endotoxin can be detected without relying on a limulus amebocyte lysate. Further, according to the present disclosure, in addition, an alternative means that is different from a conventional recombinant Factor C is realized.

Examples

The Factor C of the present disclosure is described in detail with reference to the following examples. It should be noted that these examples are provided for illustrative purposes only and the scope of the present disclosure is not limited to these examples.

[Example 1]

(1) Construction of plasmid vector for Factor C expression

(1-1) Construction of vector fragment

The pIZT/V5-His vector (Invitrogen) was prepared, 1 ng thereof was mixed with 25 μl of KOD One PCR Master Mix (TOYOBO), 1.5 μl of a 5 μM forward primer (SEQ ID NO: 1), and 1.5 μl of a 5 μM reverse primer (SEQ ID NO: 2), the amount of the mixture was adjusted to 50 μl with ultrapure water, and the resultant was subjected to PCR. PCR was performed by repeating a cycle of 98°C for 10 seconds, 55°C for 5 seconds, and 68°C for 20 seconds 25 times to amplify the fragment.

Restriction enzyme Dpnl (2 μl, New England Biolabs) was added to the PCR product, the mixture was incubated at 37°C for 1 hour, and the reaction product was purified using NucleoSpin Gel and PCR Clean-up (Macherey-Nagel) to obtain a vector fragment.

(1-2) Ligation of pIZT/V5 vector to Factor C gene

The nucleotide sequence (SEQ ID NO: 4) encoding the amino acid sequence of FC (SEQ ID NO: 3) comprising a sequence homologous to the vector added to the upstream region (SEQ ID NO: 5) and the downstream region (SEQ ID NO: 6) of ORF was synthesized contractually by Integrated DNA Technologies.

The insert fragment was lysed to 50 ng/μl with ultrapure water, 1.5 μl thereof was mixed with 1 μl of the 100 ng/μl vector fragment obtained in (1-1) and 2 μl of 5× In-Fusion Snap Assembly Master Mix (Clontech), the amount of the mixture was adjusted to 10 μl with ultrapure water, and the resultant was then subjected to a reaction at 50°C for 15 minutes. The resulting In-Fusion reaction product (5 μl) was used to transform 50 μl of ECOS™ Competent *E. coli* JM109 (Nippon Gene Co., Ltd.), and the resultant was seeded in an LB agar medium supplemented with 25 μg/ml zeocin, followed by incubation at 37°C overnight to obtain a transformant.

The resulting colonies were seeded in 2.5 ml of an LB liquid medium supplemented with 25 μg/ml zeocin and allowed to grow at 37°C and 200 rpm overnight and then, plasmids were prepared using the FastGene Plasmid Mini Kit (Nippon Genetics Co., Ltd.), and the nucleotide sequence was decoded via DNA sequencing analysis (Fasmac Co., Ltd.). It was thus confirmed that the plasmid vector for Factor C expression (pIZT/V5-FCv) was constructed. The colonies, the sequences of which had been confirmed, were seeded in 5 ml of 2TY medium supplemented with 25μg/ml zeocin and allowed to grow at 37°C and 200 rpm overnight. Thereafter, endotoxin-free plasmids were prepared using NucleoBond® Xtra Midi EF (Macherey-Nagel).

(2) Recombinant production of Factor C

(2-1) Culturing of insect cells Sf9

Sf9 cells in Sf-900™ II SFM (Thermo Fisher Scientific) were used as the insect cells, and a series of processes was performed in a safety cabinet. Sf-900™ III SFM (25 ml, Thermo Fisher Scientific) was added to a 125-ml conical flask that had been dry-heated at 250°C for 2 hours in advance, and the flask was allowed to stand in an incubator at 28°C. A Sf9-containing frozen vial was introduced into a warm bath at 37°C and rapidly thawed to retain a small amount of ice. After the vial surface was sterilized with 70% ethanol, the content of the vial was mildly mixed, and the entire amount thereof was transferred to the conical flask described above. The cap of the flask was loosened, and rotary shaking culture was performed at 28°C and 125 rpm.

Part of the cell culture liquid was sampled 3 days later and mixed with a 4% trypan blue solution at 1:1. The cell count and the viability were determined using the Countess™ Automated Cell Counter (Thermo Fisher Scientific). When the cell count was $3 \times 10^6$ to $6 \times 10^6$ cells/ml, the cells were subjected to passage culture to reach a cell count of $2 \times 10^5$ to $4 \times 10^5$ cells/ml. Passage culture was performed periodically at a frequency of once every 3 or 4 days while the present example (present experiment) was carried out.

(2-2) Transient transfection

Sf9 cells that had been subjected to passage culture 3 or more times and achieved the viability of 90% were used for transfection. Sf9 cells during culture were sampled and diluted to $4.1 \times 10^5$ cells/ml with the addition of the Sf9 cells in Sf-900™ III SFM (Thermo Fisher Scientific) brought back to room temperature. The resultant was added dropwise to a 6-well cell culture plate (Corning) at 2 ml/well, and the plate was mildly shaken alternately from side to side to uniformly distribute the cells.

The endotoxin-free plasmids obtained in the "(1) Construction of plasmid vector for Factor C expression" section above were fractionated to 1.4-μg sterilized tubes, 100 μl of Sf-900™ III SFM (Thermo Fisher Scientific) was added thereto, and the resultant was allowed to stand for 15 to 25 minutes. When doing so, Sf9 not supplemented with plasmids was prepared simultaneously as a negative control.

Subsequently, 12 µl of the Cellfectin™ II Reagent (Thermo Fisher Scientific) was mixed with 150 µl of Sf9 cells in Sf-900™ III SFM in a sample, and the resultant was allowed to stand for 15 minutes. The Cellfectin™ II Reagent was mixed thoroughly by inversion before use. The plasmids allowed to stand for a given period of time were mixed with the Cellfectin™ II Reagent, the resultants were sprinkled on each cell-containing well, the plate was mildly shaken alternately from side to side, and static culture was then performed at 28°C for 96 hours. After the completion of culturing, transfection efficiency was verified using a fluorescent microscope BZ-X810 (Keyence Corporation), and the supernatant was sampled. The supernatant was filtered through a syringe filter DISMIC 13CP020AS (Advantec Toyo Kaisha, Ltd.) to obtain an FCv culture supernatant.

(2-3) Evaluation of Factor C activity

The Sf9 culture supernatant in which FCv had been expressed transiently (10 µl) was mixed with 50 µl of an endotoxin solution (10 EU/ml), 40 µl of a 50 mM Tris-HCl (pH 8.0), and 50 µl of Otsuka distilled water (Otsuka Pharmaceutical Factory, Inc.) on the Nunc™ F96 MicroWell™ black plate (Thermo Fisher Scientific), and the mixture was heated at 37°C for 10 minutes. Thereafter, 50 µl of a fluorescent substrate was added and the fluorescence was measured using a fluorescent plate reader Infinite 200 (TECAN) set at 37°C every 5 minutes over a period of 60 minutes. Measurement was performed at an excitation wavelength of 380 nm and a fluorescence wavelength of 440 nm with a gain of 80.

The value determined using the slope function by plotting the measurement time along the X axis and the fluorescence value along the Y axis was used as the "endotoxin-dependent activity" for the following evaluation. Control samples supplemented with Otsuka distilled water instead of endotoxin were tested simultaneously, the activity determined was designated as the "endotoxin-independent activity," and samples exhibiting significantly high endotoxin-independent activity were excluded from the subsequent test. A standard endotoxin product was prepared by diluting Control Standard Endotoxin (CSE) (10 ng/vial (CAPE COD)) to 10 EU/ml with Otsuka distilled water, and a fluorescent substrate was prepared by dissolving Boc-Asp(OBzl)-Pro-Arg-MCA (Peptide Institute Inc.) using CultureSure® DMSO (FUJIFILM Wako Pure Chemical Corporation) and diluting the solution to 0.25 mM with Otsuka distilled water. All the used reagents were endotoxin-free or of biotechnology grade.

(3) Evaluation of recombinant Factor C activity

The Sf9 culture supernatant in which FCv had been expressed transiently (50 µl) was mixed with 50 µl of 100 mM Bis-Tris (pH 6.0) in a PCR tube, and the resultant was allowed to stand for 30 minutes. Thereafter, the endotoxin-dependent activity of the Factor C solution was determined in accordance with the method described above. As a result, surprisingly, recombinantly expressed FCv was capable of detecting endotoxin. With this, an alternative means that is different from conventional recombinant Factor Cs and that can be utilized for endotoxin detection was realized.

[Example 2]

Evaluation of thermostability of Factor C

Next, thermostability of FCv was evaluated. More specifically, 50 µl of the Sf9 culture supernatant in which FCv had been expressed transiently (described in Example 1) was mixed with 50 µl of 100 mM Bis-Tris (pH 6.0) in a PCR tube, the tube was mounted on the T100™ Thermal Cycler (Bio-Rad) set at 45°C, and the resultant was allowed to stand for 30 minutes. FCv was heat-treated at 45°C. The endotoxin-dependent activity of the Factor C solution after heat treatment was determined in accordance with the method described above. The activity of the sample subjected to heat treatment was divided by the activity of the sample not subjected to heat treatment, and the calculated value multiplied by 100 was designated as thermostability. As a result, FCv, which is recombinantly-expressed Factor C subjected to heat treatment at 45°C retained 67% activity and was confirmed to have a certain level of thermostability. This is considered to be usable in actual settings.

[Example 3]

Construction of plasmid vector for mutant Factor C expression

Subsequently, construction of various mutants was attempted based on FCv, which was capable of endotoxin detection.
More specifically, the pIZT/V5-FCv plasmid vector for Factor C expression constructed in Example 1 was diluted to 40 ng/µl with ultrapure water, 0.5 µl thereof was mixed with 10 µl of KOD One PCR Master Mix, 1.2 µl of a 5 µM Fw primer, and 1.2 μl of 5 μM Fw primer, the amount of the mixture was adjusted to 20 μl with ultrapure water, and the resultant was then subjected to PCR. The template plasmid vector, Fw primers, and Rv primers used for PCR are shown in the table below. PCR was performed by repeating a cycle of 98°C for 10 seconds, 55°C for 5 seconds, and 68°C for 35 seconds 15 times to amplify a fragment.

[Table 2]

| Enzyme | Enzyme before mutation | Amino acid substitution | Template plasmid vector | Fw primer | Rv primer |
|---|---|---|---|---|---|
| FCv-1 | FCv | F423L | pIZT/V5-FCv | SEQ ID NO: 7 | SEQ ID NO: 8 |
| FCv-2 | FCv | F557L | pIZT/V5-FCv | SEQ ID NO: 9 | SEQ ID NO: 10 |
| FCv-3 | FCv | F669Y | pIZT/V5-FCv | SEQ ID NO: 11 | SEQ ID NO: 12 |
| FCv-4 | FCv | L970A | pIZT/V5-FCv | SEQ ID NO: 13 | SEQ ID NO: 14 |
| FCv-5 | FCv | L970V | pIZT/V5-FCv | SEQ ID NO: 15 | SEQ ID NO: 14 |

Restriction enzyme DpnI (1 μl) was added to the PCR product, the mixture was incubated at 37°C for 1 hour, 3 ml thereof was fractionated, 30 μl of ECOS™ Competent *E. coli* JM109 was subjected to transformation, and the resultant was seeded in an LB agar medium supplemented with 25 μg/ml zeocin, followed by incubation at 37°C overnight. Thus, a transformant was obtained.

The resulting colonies were seeded in 2.5 ml of an LB liquid medium supplemented with 25 μg/ml zeocin and allowed to grow at 37°C and 200 rpm overnight. Thereafter, plasmids were prepared using the FastGene Plasmid Mini Kit, and the nucleotide sequence was examined with the use of the DNA sequencing analysis service (Fasmac Co., Ltd.). The colonies, the sequences of which had been confirmed, were seeded in 5 ml of 2TY medium supplemented with 25 μg/ml zeocin and allowed to grow at 37°C and 200 rpm overnight. Thereafter, the endotoxin-free plasmid vector for mutant FCv expression was prepared using NucleoBond® Xtra Midi EF.

With the use of the plasmid vector for mutant FCv expression obtained, insect cells were cultured in the same manner as in Example 1 and subjected to transient transfection to obtain a mutant FCv culture supernatant. Activity evaluation was also performed in the same manner as in Example 1.

(4) Evaluation of thermostability of Factor C

The Sf9 culture supernatant in which FCv or mutant FCv had been expressed transiently (50 μl) was mixed with 50 μl of 100 mM Bis-Tris (pH 6.0) in a PCR tube, the tube was mounted on the T100™ Thermal Cycler (Bio-Rad) set at 45°C, and the resultant was allowed to stand for 30 minutes. The endotoxin-dependent activity of the Factor C solution after heat treatment was determined in accordance with the method described above. The activity of the sample subjected to heat treatment was divided by the activity of the sample not subjected to heat treatment, and the calculated value multiplied by 100 was designated as thermostability. Thermostability of FCv and that of mutant FCv are shown in the table below.

[Table 3]

| Enzyme | Amino acid substitution | Thermostability (%) |
|---|---|---|
| FCv | None | 67 |
| FCv-1 | F423L | 83 |
| FCv-2 | F557L | 67 |
| FCv-3 | F669Y | 68 |
| FCv-4 | L970A | 75 |
| FCv-5 | L970V | 80 |

It was found that the amino acid substitution investigated in Example 3 contribute to improve the thermostability of the recombinant Factor C. In particular, when leucine at position 970 was substituted with alanine or valine, the FCv mutant exhibited high stability. Further, when phenylalanine at position 423 was substituted with leucine, the FCv mutant also exhibited high stability.

[Example 4]

Preparation of multiple mutant FCv

A double mutant FCv was prepared by combining the points of mutation (Table 2) contributing to the improved thermostability of FCv. A double mutant was prepared in the same manner as in Example 3. PCR was performed using the plasmid vector for FCv L970A mutant expression (pIZT/V5-FCv-4) as a template. The Fw primers and the Rv primers used are shown in the table below.

[Table 4]

| Enzyme | Amino acid substitutions accumulated in FCv | Fw primer | Rv primer |
|---|---|---|---|
| FCv-6 | L970A/F557L | SEQ ID NO: 9 | SEQ ID NO: 10 |
| FCv-7 | L970A/F669Y | SEQ ID NO: 11 | SEQ ID NO: 12 |
| FCv-8 | L970A/S973V | SEQ ID NO: 16 | SEQ ID NO: 17 |
| FCv-9 | L970A/S973I | SEQ ID NO: 18 | SEQ ID NO: 17 |
| FCv-10 | L970A/S973L | SEQ ID NO: 19 | SEQ ID NO: 17 |

Subsequently, triple and quadruple mutants FCv were prepared. Triple and quadruple mutants were prepared in the same manner as in Example 3. The Fw primers and the Rv primers used for PCR are shown in the table below. When preparing the plasmid vector for triple mutant FCv expression, the plasmid vector for FCv L970A/F557L mutant expression (pIZT/V5-FCv-6) was used as a template. When preparing the plasmid vector for quadruple mutant FCv expression, the plasmid vector for FCv L970A/F557L/F669Y mutant expression (pIZT/V5-FCv-6) was used as a template.

[Table 5]

| Enzyme | Amino acid substitutions accumulated in FCv | Fw primer | Rv primer |
|---|---|---|---|
| FCv-11 | L970A/F557L/F669Y | SEQ ID NO: 11 | SEQ ID NO: 12 |
| FCv-12 | L970A/F557L/F669Y/S973V | SEQ ID NO: 16 | SEQ ID NO: 17 |
| FCv-13 | L970A/F557L/F669Y/S973I | SEQ ID NO: 18 | SEQ ID NO: 17 |
| FCv-14 | L970A/F557L/F669Y/S973L | SEQ ID NO: 19 | SEQ ID NO: 17 |

Subsequently, a quintuple mutant FCv was prepared. A quintuple mutant was prepared in the same manner as in Example 3. The Fw primers and the Rv primers used for PCR are shown in the table below. When preparing the plasmid vector for quintuple mutant FCv expression, the plasmid vector for FCv L970A, FCv F557L, FCv F669Y, and FCv S973V mutant expression (pIZT/V5-FCv-12) was used as a template.

[Table 6]

| Enzyme | Amino acid substitutions accumulated in FCv | Fw primer | Rv primer |
|---|---|---|---|
| FCv-15 | L970A/F557L/F669Y/S973V/Y425F | SEQ ID NO: 20 | SEQ ID NO: 21 |
| FCv-16 | L970A/F557L/F669Y/S973V/Y605F | SEQ ID NO: 22 | SEQ ID NO: 23 |
| FCv-17 | L970A/F557L/F669Y/S973V/Y832F | SEQ ID NO: 24 | SEQ ID NO: 25 |
| FCv-18 | L970A/F557L/F669Y/S973V/Y833F | SEQ ID NO: 26 | SEQ ID NO: 27 |
| FCv-19 | L970A/F557L/F669Y/S973V/Y842K | SEQ ID NO: 28 | SEQ ID NO: 29 |
| FCv-20 | L970A/F557L/F669Y/S973V/A876R | SEQ ID NO: 30 | SEQ ID NO: 31 |
| FCv-21 | L970A/F557L/F669Y/S973V/K897Q | SEQ ID NO: 32 | SEQ ID NO: 33 |
| FCv-22 | L970A/F557L/F669Y/S973V/T914R | SEQ ID NO: 34 | SEQ ID NO: 35 |
| FCv-23 | L970A/F557L/F669Y/S973V/S930D | SEQ ID NO: 36 | SEQ ID NO: 37 |
| FCv-24 | L970A/F557L/F669Y/S973V/S930E | SEQ ID NO: 38 | SEQ ID NO: 37 |

(continued)

| Enzyme | Amino acid substitutions accumulated in FCv | Fw primer | Rv primer |
|---|---|---|---|
| FCv-25 | L970A/F557L/F669Y/S973V/S930K | SEQ ID NO: 39 | SEQ ID NO: 37 |
| FCv-26 | L970A/F557L/F669Y/S973V/K955E | SEQ ID NO: 40 | SEQ ID NO: 41 |
| FCv-27 | L970A/F557L/F669Y/S973V/R977E | SEQ ID NO: 42 | SEQ ID NO: 43 |
| FCv-28 | L970A/F557L/F669Y/S973V/S1013D | SEQ ID NO: 44 | SEQ ID NO: 45 |

(5) Evaluation of thermostability of multiple mutant FCv

The results of evaluation of thermostability of the double mutants are shown in the table below. Evaluation of thermostability was performed in accordance with the method described in the "(4) Evaluation of thermostability of Factor C" section. Heat treatment was performed at 45°C. All double mutants were confirmed to have thermostability superior to that of a single mutant.

[Table 7]

| Enzyme | Accumulated amino acid substitutions | Thermostability (%) |
|---|---|---|
| FCv | None | 67 |
| FCv-5 | L970V | 80 |
| FCv-6 | L970A/F557L | 95 |
| FCv-7 | L970A/F669Y | 99 |
| FCv-8 | L970A/S973V | 90 |
| FCv-9 | L970A/S973I | 92 |
| FCv-10 | L970A/S973L | 90 |

The results of evaluation of thermostability of the triple and quadruple mutants are shown in the table below. Evaluation of thermostability was performed in accordance with the method described in the "(4) Evaluation of thermostability of Factor C" section. Since triple and quadruple mutants had high stability and thermostability of all the mutants did not fall below 100% even when treated at 45°C, heat treatment was performed at 48°C. All the triple mutants and the quadruple mutants exhibited the improved thermostability.

[Table 8]

| Enzyme | Amino acid substitutions accumulated in FCv | Thermostability (%) |
|---|---|---|
| FCv | None | 3 |
| FCv-11 | L970A/F557L/F669Y | 50 |
| FCv-12 | L970A/F557L/F669Y/S973V | 77 |
| FCv-13 | L970A/F557L/F669Y/S973I | 111 |
| FCv-14 | L970A/F557L/F669Y/S973L | 82 |

The results of evaluation of thermostability of the quintuple mutants are shown in the table below. Evaluation of thermostability was performed in accordance with the method described in the "(4) Evaluation of thermostability of Factor C" section. Heat treatment was performed at 48°C. All the quintuple mutants were confirmed to have improved thermostability. This indicates that a multiple mutant may be prepared using one of the amino acid substitutions contributing to the improved thermostability found herein or a plurality thereof in combination, so as to obtain recombinant Factor C with significantly improved thermostability.

[Table 9]

| Enzyme | Accumulated amino acid substitutions | Thermostability (%) |
|---|---|---|
| FCv-12 | L970A/F557L/F669Y/S973V | 77 |
| FCv-15 | L970A/F557L/F669Y/S973V/Y425F | 99 |
| FCv-16 | L970A/F557L/F669Y/S973V/Y605F | 84 |
| FCv-17 | L970A/F557L/F669Y/S973V/Y832F | 97 |
| FCv-18 | L970A/F557L/F669Y/S973V/Y833F | 92 |
| FCv-19 | L970A/F557L/F669Y/S973V/Y842K | 83 |
| FCv-20 | L970A/F557L/F669Y/S973V/A876R | 102 |
| FCv-21 | L970A/F557L/F669Y/S973V/K897Q | 104 |
| FCv-22 | L970A/F557L/F669Y/S973V/T914R | 79 |
| FCv-23 | L970A/F557L/F669Y/S973V/S930D | 90 |
| FCv-24 | L970A/F557L/F669Y/S973V/S930E | 83 |
| FCv-25 | L970A/F557L/F669Y/S973V/S930K | 113 |
| FCv-27 | L970A/F557L/F669Y/S973V/R977E | 81 |
| FCv-28 | L970A/F557L/F669Y/S973V/S1013D | 92 |

Subsequently, sextuple to nonuple mutants FCv were prepared. Multiple mutants were prepared in the same manner as in Example 3. The Fw primers and the Rv primers used for PCR are shown in the table below. The plasmid vector for sextuple mutant FCv expression was prepared using the plasmid vector for FCv L970A, F557L, F669Y, S973V, and Y842K mutant expression (pIZT/V5-FCv-19) as a template. The plasmid vector for septuple mutant FCv expression was prepared using the plasmid vector for FCv L970A, F557L, F669Y, S973V, Y842K, and A876R mutant expression (pIZT/V5-FCv-29) as a template. The plasmid vector for octuple mutant FCv expression was prepared using the plasmid vector for FCv L970A, F557L, F669Y, S973V, Y842K, A876R, and R977E mutant expression (pIZT/V5-FCv-30) as a template. The plasmid vector for nonuple mutant FCv expression was prepared using the plasmid vector for FCv L970A, F557L, F669Y, S973V, Y842K, A876R, R977E, and S1013D mutant expression (pIZT/V5-FCv-31) as a template.

[Table 10]

| Enzyme | Amino acid substitutions accumulated in FCv | Fw primer | Rv primer |
|---|---|---|---|
| FCv-19 | L970A/F557L/F669Y/S973V/Y842K | SEQ ID NO: 28 | SEQ ID NO: 29 |
| FCv-29 | L970A/F557L/F669Y/S973V/Y842K/A876R | SEQ ID NO: 30 | SEQ ID NO: 31 |
| FCv-30 | L970A/F557L/F669Y/S973V/Y842K/A876R/R977E | SEQ ID NO: 42 | SEQ ID NO: 43 |
| FCv-31 | L970A/F557L/F669Y/S973V/Y842K/A876R/R977E/S1013D | SEQ ID NO: 44 | SEQ ID NO: 45 |
| FCv-32 | L970A/F557L/F669Y/S973V/Y842K/A876R/R977E/S1013D/K897Q | SEQ ID NO: 32 | SEQ ID NO: 33 |
| FCv-33 | L970A/F557L/F669Y/S973V/Y842K/A876R/R977E/S1013D/S930E | SEQ ID NO: 38 | SEQ ID NO: 37 |
| FCv-34 | L970A/F557L/F669Y/S973V/Y842K/A876R/R977E/S1013D/S930K | SEQ ID NO: 39 | SEQ ID NO: 37 |
| FCv-35 | L970A/F557L/F669Y/S973V/Y842K/A876R/R977E/S1013D/K955E | SEQ ID NO: 40 | SEQ ID NO: 41 |

The results of evaluation of thermostability of multiple mutants are shown in the table below. Evaluation of thermostability was performed in accordance with the method described in the "(4) Evaluation of thermostability of Factor C" section. Stability of the sextuple to nonuple mutants was superior to that of the quintuple mutant and thermostability thereof exceeded 100% even when treated at 48°C and, therefore, heat treatment was performed at 49°C. All the multiple mutants exhibited improved thermostability. By accumulating the sites of mutation from the quintuple mutation to the nonuple mutation, more surprisingly, Factor C with thermostability improved to a significant extent was obtained.

[Table 11]

| Enzyme | Amino acid substitutions accumulated in FCv | Thermostability (%) |
|--------|---------------------------------------------|---------------------|
| FCv-19 | L970A/F557L/F669Y/S973V/Y842K | 63 |
| FCv-29 | L970A/F557L/F669Y/S973V/Y842K/A876R | 70 |
| FCv-30 | L970A/F557L/F669Y/S973V/Y842K/A876R/R977E | 76 |
| FCv-31 | L970A/F557L/F669Y/S973V/Y842K/A876R/R977E/S1013D | 77 |
| FCv-32 | L970A/F557L/F669Y/5973 V/Y842K/A876R/R977E/S1013D/K897Q | 121 |
| FCv-33 | L970A/F557L/F669Y/S973V/Y842K/A876R/R977E/S1013D/S930E | 103 |
| FCv-34 | L970A/F557L/F669Y/S973V/Y842K/A876R/R977E/S1013D/S930K | 81 |
| FCv-35 | L970A/F557L/F669Y/S973V/Y842K/A876R/R977E/S1013D/K955E | 94 |

As described above, one of the amino acid substitutions contributing to improve the thermostability discovered herein may be used to prepare a single mutant or such amino acid substitutions may be used in combination of two or more to prepare a multiple mutant. Thus, a recombinant Factor C with significantly improved thermostability can be obtained.

Industrial applicability

The recombinant protein(s) of the present disclosure can be used for a variety of applications as a protease. For example, the recombinant protein of the present disclosure can be used for endotoxin measurement, endotoxin detection, or other applications, although the applications are not limited thereto.

The present specification cites many documents including patent applications, academic literatures, and manufacturers' instructions. While the disclosures of such documents are not regarded as being related to patentability of the present invention, such disclosures are incorporated herein by reference in their entirety. More specifically, all of the reference documents are incorporated herein by reference as in the case in which each of the documents is specifically and individually indicated to be incorporated herein by reference.

Brief Description of Sequences

SEQ ID NOs: 1 and 2: primers for amplifying the pIZT/V5 fragment
SEQ ID NO: 3: the amino acid sequence of FCv
SEQ ID NO: 4: the nucleotide sequence of the FCv gene
SEQ ID NO: 5: homologous sequence added to upstream of the initiation codon
SEQ ID NO: 6: homologous sequence added to downstream of the termination codon
SEQ ID NOs: 7 to 45: primers for introducing FCv mutation

Sequences

SEQ ID NO: 1: forward primer
tgagtttatctgactaaatcttag
SEQ ID NO: 2: reverse primer
ggtaccaagctttaaattcgaacag
SEQ ID NO: 3: the amino acid sequence of FCv

MVLASFLVSGLVLGLLAQQMHPVQSRGVDLGLCDDTRFECKCGDPGYVFNIPVKQC

TYFYRWRPYCKPCDKLEAKDVCPKYKRCQECRAGLDSCVGCPPNKYGTWCSGECQ

CKNGGICDQRTGACACRDRYEGLHCEILRGCPLLNSDTQVQEVKNPPQDPQTIDYSC

PPGFKLKGVARINCLPNGQWSSFPPKCIRECSMVSSLEHGKVTSLSAELIEGATLRFSC

DSPHYLIGQETLTCQGNGQWSGQIPQCQKLVFCPDLDPVSHAEHKVKIGLEQKYGQF

PQGTEVTYTCTGNYFLMGLDTLKCNLDGSWSGTQPSCVKVADREVSCDSKAVDFLD

DVGEPVRIHCPAGCSLTAGTVWGTAIYHELSSVCRAAIHAGKVPNSGGAVHVVNNG

PYSDFLASDLNGIKSDELKSLAQSFRFDYVRSSTAGKSGCPDGWFEIDENCVYVTSKQ

RAWERAQGVCTNMAARLAVLDKDVIPSSLTETLRGKGLSTTWIGLHRLDADNHFLW

ELMDRSTVALNDQLTFWAPGEPGNETNCVYMDIQDQLQSVWKTKSCFQPSSFVCM

MDLSDKNKAKCEDPGPLENGHAKLHGQSIDGFYAGSSVRYSCEVLHYLSGTETVSCT

SNGTWSAPKPRCIKVITCQTPPVPSYGSVDIKPPSRTNSISRVGSPFLRLPRLPLPLARA

AGPPPKPRSAPPSTVDLSSKVKLPEGHYRVGSQAIYTCESRYYELLGSQGRRCDSNGK

WSGRPASCIPVCGRSDSPRSPFIVNGNSTEIGQWPWQAGISRWLADHNMWFLLCGGA

LLNEKWIITAAHCVTYSATAEIIDPSQFKFYLGKYYRDDSKDDDYVQVRTAQEIHVNP

NYDPGRLDADIALIQLEESVALTTRVQPICLPTDRSSRNNLKEGHKAVVTGWGLNEN

ETYSEMIQQAVLPVVSASTCEQGYRSSGVPLTVTENMFCAGYKNGRYDSCSGDSGGP

LVFSDESRTDRRWVLEGIISWGSVNGCGKANQYAGFTKVNVFLSWIRQFI

SEQ ID NO: 4: the nucleotide sequence of the FCv gene

atggtgctggctagcttcctggtgtctggactggtgctgggactgctggctcagcaaatgcaccctgtgcagtctcgtggtgtcgacctg

ggattgtgcgacgacactcgtttcgagtgcaagtgcggcgaccccggttacgtgttcaacatcccagtgaagcagtgcacctacttcta

ccgttggcgcccctactgcaagccctgcgacaagttggaagctaaggacgtgtgccccaagtacaagcgttgccaagagtgccgtgc

tggcctggactcctgtgttggttgccctcctaacaaatacggcacctggtgctctggcgagtgccagtgcaagaacggtggtatctgcg

accagcgtaccggtgcttgcgcttgccgtgatcgttacgagggtctgcactgcgagatcctgcgtggttgccctctgctgaactctgaca

cccaggtgcaagaagtgaagaaccctccacaggaccctcagaccatcgactactcttgccctcccggtttcaagctgaagggtgtcgc

tcgtatcaactgcctgcctaacggccagtggtctagcttcccacctaagtgcatccgcgagtgctccatggtgtcctcactcgagcacgg

caaagtgacctctctgtccgctgagctgatcgagggtgctaccttgaggttctcctgcgactcccctcactacctgatcggtcaagagac

tctgacctgccaaggcaacggacagtggagcggtcaaatccctcagtgccaaaagctggtgttctgccccgacttggaccccgtgtct

cacgctgaacacaaggtgaagatcggcctcgagcagaagtacggacagttccctcagggcaccgaagtgacctacacctgtaccgg

caactacttcctgatgggcctcgacaccctgaagtgcaacctggacggttcttggagcggtactcagccctcctgcgtgaaagtggctg

accgtgaagtgtcctgcgactctaaggctgtggacttcctggacgacgtgggagagcctgtgcgtattcactgtcctgctggttgctccc

tgaccgctggtactgtttgggggcaccgctatctaccacgagctgtcctctgtgtgccgcgctgctattcacgctggaaaggtgcccaact

ccggtggtgctgtgcacgtggtcaacaacggtccctactccgacttcctcgcttccgacctgaacggtatcaagtccgacgagctgaag

tccctggctcagtccttccgtttcgactacgtgcgttcctccaccgctggaaagtctggttgtcctgacggatggttcgagatcgacgaga

actgcgtgtacgtgacctccaagcagagagcttgggaacgcgctcaaggcgtgtgcaccaatatggctgctcgtctggctgtgctgga

caaggacgtgatcccttccagcctgactgagactctgcgtggaaagggactctccaccacctggatcggactgcaccgtttggacgct

gacaaccacttcctgtgggagctgatggaccgttccaccgtggctctgaacgaccagctgactttctgggctcctggcgagcctggca

acgagactaactgcgtctacatggacatccaggaccagctgcagtccgtgtggaaaactaagtcctgcttccagccatcctccttcgtgt

gcatgatggacctgtccgacaagaacaaggctaagtgcgaggaccccggtcctctcgagaacggtcacgctaagctgcacggccag

tccatcgacggtttctacgctggttcctctgtgcgctactcctgcgaggtgctgcactacctgtccggaaccgagactgtgtcctgcacct

ccaacggaacttggagcgcacctaagcctcgttgcatcaaagtgatcacctgtcagacccctccagtgccttcctacggttccgtggac

atcaagccaccttctcgtaccaactccatctctcgtgtgtgggttctcccttcctgcgtctgcctcgtttgcctctgcctttggctagagctgctg

gtccacctcctaagccacgttccgctcctccatctaccgtggacttgtcctccaaagtgaagctgcccgagggccactaccgtgtcggtt

cccaggctatctacacttgcgagtcccgctactacgaactgctgggttctcagggtcgtcgttgcgactcaaatggcaagtggagtggtc

gtcccgcttcttgcatccctgtgtgcggtcgttctgactcccctcgttctcctttcatcgtgaacggcaactccaccgagatcggccaatgg

ccttggcaagctggtatctccagatggctggctgaccacaacatgtggttcttgctgtgcggtggcgctctgctgaacgagaagtggatc

atcaccgctgctcactgcgtgacctactccgctaccgctgagatcatcgacccctctcagttcaagttctacctgggcaagtactaccgtg

acgactccaaggacgacgactacgtccaagtgcgcaccgctcaggagatccacgtgaaccctaactacgaccccggccgtctggac

gctgatatcgctctgatccagctcgaggagtctgtcgctctgaccaccagggtgcagcctatctgcttgcctaccgaccgttcctcccgc

aacaacctgaaagagggtcacaaggctgtcgtgaccggatggggactcaacgagaacgagacctactctgagatgatccagcaggc

cgtgctgccagtggtgtccgcttctacttgcgagcagggttaccgttcctccggagtgcctctgactgtgaccgaaaacatgttctgcgct

ggttacaagaacggccgttacgactcctgctcaggcgattctggtggtcccctggtgttctccgacgagtctcgtactgatcgtcgttgg

gtgctcgagggtatcatctcctggggttctgtgaacggttgcggcaaggctaaccagtacgctggttttaccaaagtcaacgtgttcctgt

cctggatccgtcagttcatctga

SEQ ID NO: 5: homologous sequence added to upstream of the initiation codon
ctgttcgaatttaaagcttggtacc
SEQ ID NO: 6: homologous sequence added to downstream of the termination codon
tgagtttatctgactaaatcttag
SEQ ID NO: 7: Fw primer for introducing FCv F423L mutation
cagtccttccgtctcgactacgtgcgttcc
SEQ ID NO: 8: Rv primer for introducing FCv F423L mutation
acggaaggactgagccagggacttcagctc
SEQ ID NO: 9: Fw primer for introducing FCv F557L mutation

actaagtcctgcctccagccatcctccttc

SEQ ID NO: 10: Rv primer for introducing FCv F557L mutation

gcaggacttagtttttccacacggactgcag

SEQ ID NO: 11: Fw primer for introducing FCv F669Y mutation

gtgggttctccctacctgcgtctgcctcgt

SEQ ID NO: 12: Rv primer for introducing FCv F669Y mutation

gggagaacccacacgagagatggagttggt

SEQ ID NO: 13: Fw primer for introducing FCv L970A mutation

tctggtggtcccgcggtgttctccgacgag

SEQ ID NO: 14: Rv primer for introducing FCv L970A and FCv L970V mutations

gggaccaccagaatcgcctgagcaggagtc

SEQ ID NO: 15: Fw primer for introducing FCv F970V mutation

tctggtggtcccgtggtgttctccgacgag

SEQ ID NO: 16: Fw primer for introducing FCv S973V mutation

cccgcggtgttcgtcgacgagtctcgtact

SEQ ID NO: 17: Rv primer for introducing FCv S973V, FCv S973I, and FCv S973L mutations gaacaccgcgggacca ccagaatcgcctga

SEQ ID NO: 18: Fw primer for introducing FCv S973I mutation

Cccgcggtgttcatcgacgagtctcgtact

SEQ ID NO: 19: Fw primer for introducing FCv S973L mutation

cccgcggtgttcttagacgagtctcgtact

SEQ ID NO: 20: Fw primer for introducing FCv Y425F mutation

ttccgtttcgacttcgtgcgttcctccacc

SEQ ID NO: 21: Rv primer for introducing FCv Y425F mutation

gtcgaaacggaaggactgagccagggactt

SEQ ID NO: 22: Fw primer for introducing FCv Y605F mutation

tcctctgtgcgcttctcctgcgaggtgctg

SEQ ID NO: 23: Rv primer for introducing FCv Y605F mutation

gcgcacagaggaaccagcgtagaaaccgtc

SEQ ID NO: 24: Fw primer for introducing FCv Y832F mutation

tacctgggcaagttctaccgtgacgactcc

SEQ ID NO: 25: Rv primer for introducing FCv Y832F mutation

cttgcccaggtagaacttgaactgagaggg

SEQ ID NO: 26: Fw primer for introducing FCv Y833F mutation

ctgggcaagtacttccgtgacgactccaag

SEQ ID NO: 27: Rv primer for introducing FCv Y833F mutation

gtacttgcccaggtagaacttgaactgaga

SEQ ID NO: 28: Fw primer for introducing FCv Y842K mutation

aaggacgacgacaaagtccaagtgcgcacc

SEQ ID NO: 29: Rv primer for introducing FCv Y842K mutation

gtcgtcgtccttggagtcgtcacggtagta

SEQ ID NO: 30: Fw primer for introducing FCv A876R mutation

gaggagtctgtccgtctgaccaccagggtg

SEQ ID NO: 31: Rv primer for introducing FCv A876R mutation

gacagactcctcgagctggatcagagcgat

SEQ ID NO: 32: Fw primer for introducing FCv K897Q mutation

cgcaacaacctgcaagagggtcacaaggct

SEQ ID NO: 33: Rv primer for introducing FCv K897Q mutation

caggttgttgcgggaggaacggtcggtagg

SEQ ID NO: 34: Fw primer for introducing FCv T914R mutation

aacgagaacgagcgctactctgagatgatc

SEQ ID NO: 35: Rv primer for introducing FCv T914R mutation

ctcgttctcgttgagtccccatccggtcac

SEQ ID NO: 36: Fw primer for introducing FCv S930D mutation

gtggtgtccgctgatacttgcgagcagggt

SEQ ID NO: 37: Rv primer for introducing FCv S930D, S930E, and S930K mutations

agcggacaccactggcagcacggcctgctg

SEQ ID NO: 38: Fw primer for introducing FCv S930E mutation

gtggtgtccgctgaaacttgcgagcagggt
SEQ ID NO: 39: Fw primer for introducing FCv S930K mutation
gtggtgtccgctaaaacttgcgagcagggt
SEQ ID NO: 40: Fw primer for introducing FCv K955E mutation
tgcgctggttacgagaacggccgttacgac
SEQ ID NO: 41: Rv primer for introducing FCv K955E mutation
gtaaccagcgcagaacatgttttcggtcac
SEQ ID NO: 42: Fw primer for introducing FCv K977E mutation
gtcgacgagtctgaaactgatcgtcgttgg
SEQ ID NO: 43: Rv primer for introducing FCv K977E mutation
agactcgtcgacgaacaccgcgggaccacc
SEQ ID NO: 44: Fw primer for introducing FCv S1013D mutation
aacgtgttcctggactggatccgtcagttc
SEQ ID NO: 45: Rv primer for introducing FCv S1013D mutation
caggaacacgttgactttggtaaaaccagc

**Claims**

1. A Factor C protein having 95% or higher amino acid sequence identity to SEQ ID NO: 3.

2. A polynucleotide encoding the protein of claim 1.

3. A reagent for detecting endotoxin comprising the protein of claim 1.

4. A cell comprising the polynucleotide of claim 2.

5. A method for producing a Factor C protein comprising culturing the cell of claim 4 and obtaining the Factor C protein of claim 1.

6. A method for producing a reagent for detecting endotoxin comprising culturing the cell of claim 4 and obtaining the Factor C protein of claim 1.

7. A method for detecting endotoxin comprising a step of bringing the protein of claim 1 or the reagent of claim 3 into contact with a sample and a step of detecting endotoxin.

# Fig. 1

Endotoxin

LAL reagent

Factor C → Activated Factor C

Factor B → Activated Factor B

Proclotting enzyme → Clotting enzyme

Coagulogen → Coagulin (coagulation)

Coagulation/turbidimetry

Peptide-label → Label (coloring)

Colorimetry

Peptide-label → Label (fluorescent)

Fluorometry

## INTERNATIONAL SEARCH REPORT

| International application No. |
| --- |
| **PCT/JP2024/012571** |

| | |
| --- | --- |
| **A.** | **CLASSIFICATION OF SUBJECT MATTER** |

*C12N 15/57*(2006.01)i; *C12N 1/15*(2006.01)i; *C12N 1/19*(2006.01)i; *C12N 1/21*(2006.01)i; *C12N 5/10*(2006.01)i; *C12N 9/48*(2006.01)i; *C12Q 1/37*(2006.01)i
FI:  C12N15/57; C12N1/15; C12N1/19; C12N1/21; C12N5/10; C12N9/48 ZNA; C12Q1/37

According to International Patent Classification (IPC) or to both national classification and IPC

| | |
| --- | --- |
| **B.** | **FIELDS SEARCHED** |

Minimum documentation searched (classification system followed by classification symbols)

C12N15/57; C12N1/15; C12N1/19; C12N1/21; C12N5/10; C12N9/48; C12Q1/37

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)GenBank/EMBL/DDBJ/GeneSeq; UniProt/GeneSeq

| | |
| --- | --- |
| **C.** | **DOCUMENTS CONSIDERED TO BE RELEVANT** |

| Category* | Citation of document, with indication, where appropriate, of the relevant passages | Relevant to claim No. |
| --- | --- | --- |
| X | WO 2018/074498 A1 (SEIKAGAKU CORPORATION) 26 April 2018 (2018-04-26) claims, paragraph [0173], examples | 1-7 |
| X | MALONEY, Tom et al. Saving the horseshoe crab: A synthetic alternative to horseshoe crab blood for endotoxin detection. PLOS Biology. 12 October 2018, vol. 16, no. 10, e2006607, DOI: 10.1371/journal.pbio.2006607 abstract, p. 3, 3rd paragraph to p. 6, 3rd paragraph | 1-7 |

☐ Further documents are listed in the continuation of Box C.　　☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **27 May 2024** | **04 June 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT

International application No.

**PCT/JP2024/012571**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/JP2024/012571**

| Patent document cited in search report | | | Publication date (day/month/year) | Patent family member(s) | | | Publication date (day/month/year) |
|---|---|---|---|---|---|---|---|
| WO | 2018/074498 | A1 | 26 April 2018 | US | 2019/0241629 | A1 | |
| | | | | claims, paragraph [0347], examples | | | |
| | | | | JP | 18-74498 | A1 | |
| | | | | EP | 3530670 | A1 | |
| | | | | CN | 109689681 | A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 1995014931 A
- JP 2009150903 A
- WO 2018074498 A
- JP 6927993 B
- US 5712144 A
- US 5716834 A
- US 5858706 A
- US 5985590 A
- US 6645724 B
- WO 2008004674 A
- JP 2014510898 A
- WO 2014092079 A
- JP 2023055723 A

### Non-patent literature cited in the description

- *PLoS Biol.*, October 2018, vol. 16 (10), e2006607
- *Chem. Rev.*, 2005, vol. 105 (11), 4056-72
- *Comb. Chem. High Throughput Screen.*, 2008, vol. 11 (2), 127-34
- *Curr. Opin. Struct. Biol.*, 2007, vol. 17, 474-80
- **AGRESTI et al.** Ultrahigh-throughput screening in drop-based microfluidics for directed evolution. *Proceedings of the National Academy of Sciences*, March 2010, vol. 107 (9), 4004-4009
- **STRYER et al.** *Biochemistry*, 2002, vol. 5, 44-49
- **S. HEINKOFF** ; **J. G. HENIKOFF**. *Proc. Natl. Acad. Sci.*, 1992, vol. 89, 10915-10919
- **THOMPSON**. *Nucleic Acid Research*, 1994, vol. 22 (22), 4673-4680